# EUROPEAN PATENT SPECIFICATION

(11) **EP 4 082 082 B1**
(45) Date of publication and mention of the grant of the patent: **28.01.2026**
(21) Application number: 20904437.9
(22) Date of filing: 22.12.2020
(51) Int. Cl.: A61M 16/00

(54) **RESPIRATORY THERAPY APPARATUS WITH REMOVABLE CONNECTIVITY MODULE AND COMPONENTS THEREOF**
ATEMTHERAPIEGERÄT MIT ABNEHMBAREM ANSCHLUSSMODUL UND KOMPONENTEN DAFÜR
APPAREIL DE THÉRAPIE RESPIRATOIRE AVEC MODULE DE CONNECTIVITÉ AMOVIBLE ET SES COMPOSANTS

(30) Priority: 24.12.2019 AU 2019904934
(43) Date of publication of application: 02.11.2022
(73) Proprietor: ResMed Pty Ltd, Bella Vista, New South Wales 2153 (AU)
(72) Inventor: AUSTIN, Benjamin, Matthew, Bella Vista, New South Wales 2153 (AU); LEAVENS, Benjamin, John, Bella Vista, New South Wales 2153 (AU); WU, Nan, Hai, Bella Vista, New South Wales 2153 (AU)
(74) Representative: Vossius & Partner Patentanwälte Rechtsanwälte mbB
(86) International application number: PCT/AU2020/051411
(87) International publication number: WO 2021/127727

(56) References cited:
- EP-B1- 2 445 082
- EP-B1- 2 572 747
- US-A1- 2007 123 065
- US-A1- 2007 169 776
- US-A1- 2011 259 334
- US-A1- 2013 239 960
- US-A1- 2016 158 478
- US-A1- 2017 151 411
- US-B2- 9 512 856
- US-B2- 9 943 269

## Description

### 1 CROSS-REFERENCE TO RELATED APPLICATIONS

Not Applicable

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. The present technology also relates to respiratory therapy devices or apparatus with removable connectivity module(s) and components thereof.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), High Flow Therapy (HFT), and long-term oxygen therapy (LTOT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep, NIV or IV form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series Tango^{™} | 31.9 | 2007 |
| C-Series Tango^{™} with Humidifier | 33.1 | 2007 |
| S8 Escape^{™} II | 30.5 | 2005 |
| S8 Escape^{™} II with H4i^{™} Humidifier | 31.1 | 2005 |
| S9 AutoSet^{™} | 26.5 | 2010 |
| S9 AutoSet^{™} with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD. Other examples of ventilators include the ResMed Lumis^{™} Series of non-invasive ventilators and the ResMed Astral^{™} series of life support ventilators.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters. US2013/239960 A1 discloses an apparatus for treatment of respiratory conditions such as pressure treatment apparatus for conditions related to obstructive sleep apnea (OSA), central sleep apnea (CSA), sleep disordered breathing (SDB), Cheyne-Stokes respiration (CSR) allergy induced upper airway obstruction, early viral infection of the upper airway, respiratory insufficiency etc.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air. Humidifiers therefore often have the capacity to heat the flow of air was well as humidifying it.

A range of artificial humidification devices and systems are known; however, they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification; some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability. The invention is defined by the appended claims.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of one form of the present technology is to provide an apparatus, or system, for supplying respiratory therapy comprising one or more removable connectivity modules, each removable connectivity module being configured to enable electrical and/or mechanical connection to one or more other systems or devices. The apparatus, or system, may comprise a connector assembly to which each of the removable connectivity modules may be configured to connect. The connectivity modules may be interchanged according to the desired connections for a particular application of use of the apparatus or system.

An aspect of one form of the present technology is to provide a removable connectivity module for a respiratory therapy device.

An aspect of one form of the present technology is an apparatus for supplying a flow of breathable gas at a positive pressure for respiratory therapy, the apparatus comprising:
a pressure generator for generating the flow of breathable gas and supplying the flow to an outlet;
a housing which contains at least the pressure generator; and
at least one electrical connector,

wherein the housing comprises a cavity configured to receive a removable connectivity module, and
wherein the at least one electrical connector is configured to connect to the connectivity module in use.

In examples, the cavity may be located in a rear of the housing.

In examples, the cavity may comprise an indexing feature to ensure that the connectivity module can only be inserted into the cavity in a single orientation.

In examples, the indexing feature may be a chamfered corner of the cavity.

In examples, the apparatus may further comprise at least one attachment mechanism configured to selectively secure the connectivity module, at least partially within the cavity.

In examples, the attachment mechanism may comprise one or more of clips, fasteners, protrusions, apertures or magnets.

In examples, the apparatus may further comprise a connector assembly, wherein the connector assembly is fixed to the housing. In examples the cavity may be provided in the connector assembly. In examples the connectivity module may be configured to connect, in use, to the connector assembly.

In examples the connector assembly may comprise at least one power connector.

In examples the cavity may comprise a slot configured to receive an interfacing connector.

In examples, the interfacing connector may pass through the slot and connect to the at least one electrical connector.

In examples, the cavity may have a depth of between approximately 15mm and approximately 35mm. For example, between approximately 20mm and approximately 30mm. For example, approximately 25mm.

In examples, the cavity may have a height of between approximately 50mm and approximately 80mm. For example, between approximately 60mm and approximately 70mm. For example, approximately 65mm.

In examples, cavity may have a width of between approximately 75mm and approximately 100mm. For example, between approximately 85mm and approximately 95mm. For example, approximately 90mm.

In examples, the apparatus may comprise a protective cover configured to cover at least one electrical connector in use.

Another aspect of one form of the present technology is to provide a connectivity module for use in a respiratory therapy apparatus, the connectivity module comprising:
a housing which comprises a front side and a rear side; and
at least one electrical connector on the front side, and at least one electrical connector on the rear side;

wherein the housing is configured to be at least partially inserted into a cavity in a respiratory therapy apparatus in use, and
wherein the at least one electrical connector on the rear side is configured to connect to at least one complementary electrical connector on the respiratory therapy apparatus.

In examples, the connectivity module may further comprise at least one attachment mechanism to secure the connectivity module at least partially within the cavity of the RPT device in use.

In examples, the at least one attachment mechanism may comprise at least one of: clips, fasteners, protrusions, apertures or magnets.

In examples, the connectivity module may further comprise an indexing feature structured to ensure that the connectivity module can only be inserted into the cavity in a single orientation.

In examples, the indexing feature may be a chamfered corner.

In examples, the at least one electrical connector on the front side may comprise at least one of: a USB connector, a d-subminiature connector, an ethernet connector, an SpO2 sensor connector and/or a remote alarm connector.

In examples, the connectivity module may further comprise a protective cover configured to selectively cover the at least one electrical connector on the front side.

In examples, the housing may comprise a shell and a backplate.

In examples, the shell may be ultrasonically welded to the backplate.

In examples, the at least one connector on the rear side may comprise edge contacts.

In examples, the connectivity module may further comprise an electronic printed circuit board (PCB).

In examples, the electronic PCB may comprise an output device in the form of a light emitting diode.

In examples, the electronic PCB may comprise one or more identification components which enable the RPT device to detect and/or identify the connectivity module.

In examples, the connectivity module may have a thickness of between approximately 15mm and approximately 35mm. For example, between approximately 20mm and approximately 30mm. For example, approximately 25mm.

In examples, the connectivity module may have a height of between approximately 50mm and approximately 80mm. For example, between approximately 60mm and approximately 70mm. For example, approximately 65mm.

In examples, the connectivity module may have a width of between approximately 75mm and approximately 100mm. For example, between approximately 85mm and approximately 95mm. For example, approximately 90mm.

Another aspect of one form of the present technology is system for providing respiratory therapy comprising:
an apparatus as described herein; and
at least one connectivity module as described herein.

In examples, the connectivity module is a first connectivity module and the system further comprises a second connectivity module as described herein, wherein the first connectivity module and the second connectivity module are interchangeably connectable to the apparatus, and wherein the first connectivity module comprises a first set of electrical connectors and the second connectivity module comprises a second set of electrical connectors, wherein the first set of electrical connectors is different to the second set of electrical connectors.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

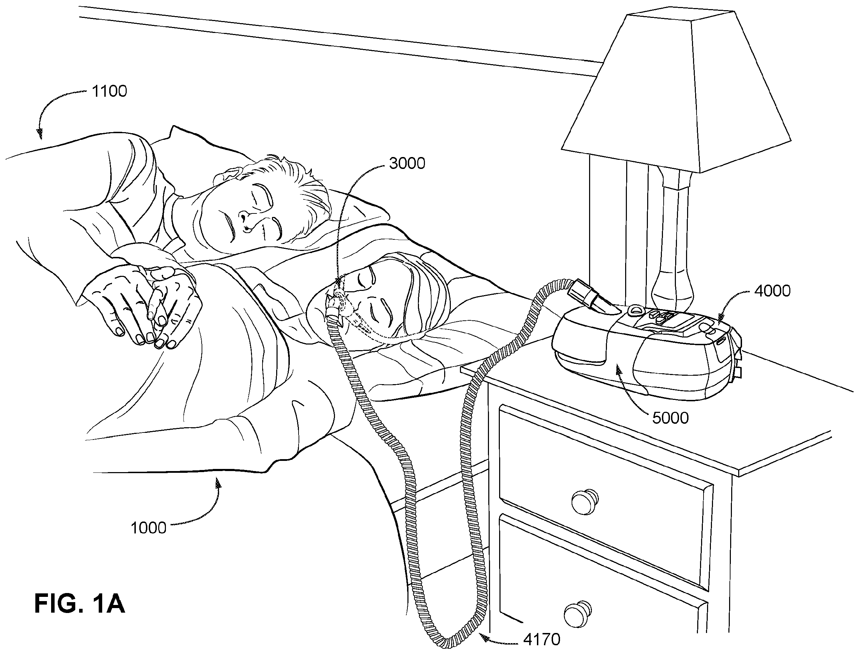

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

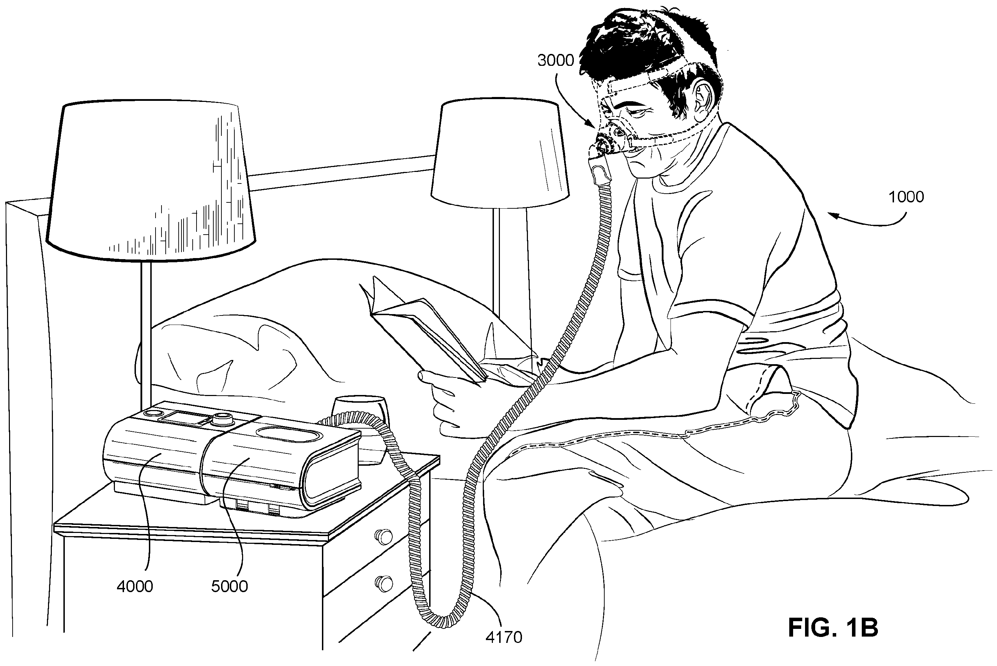

Fig. 1B shows a system including a patient 1000 wearing a patient interface 3000, in the form of a nasal mask. receiving a supply of air at positive pressure from an RPT device 4000. Air from the RPT device is humidified in a humidifier 5000, and passes along an air circuit 4170 to the patient 1000.

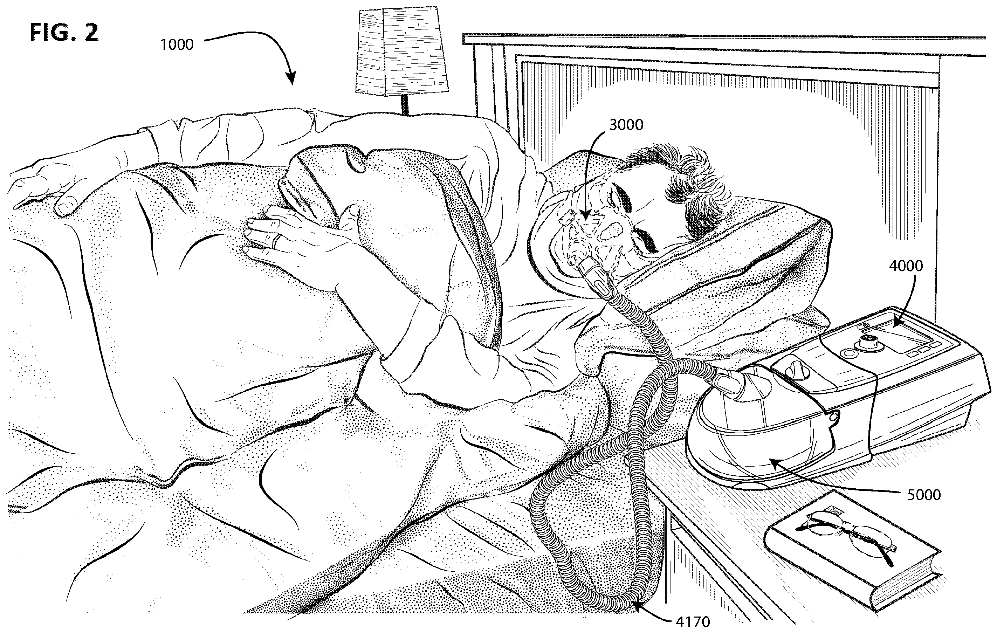

Fig. 2 shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 PATIENT INTERFACE

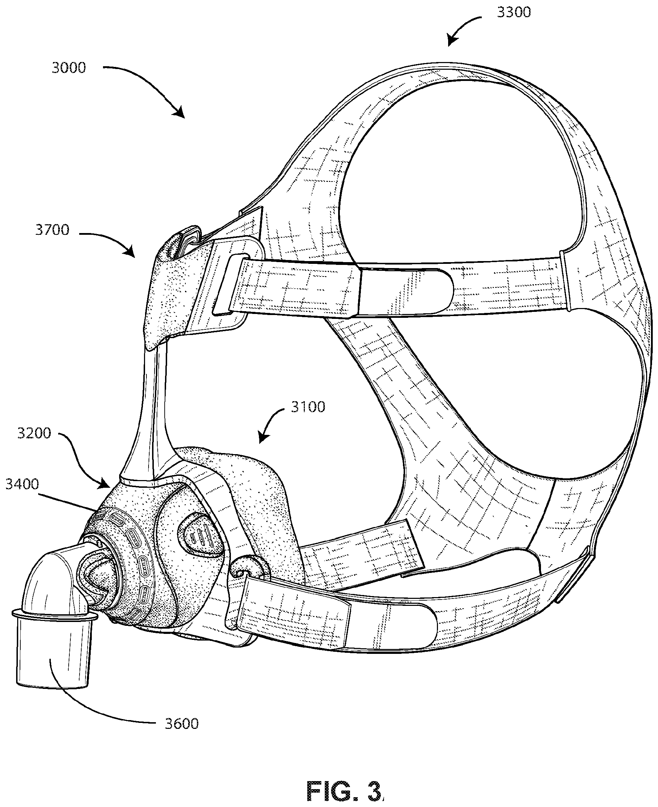

Fig. 3 shows a patient interface in the form of a nasal mask in accordance with one form of the present technology.

### 4.3 RPT DEVICE

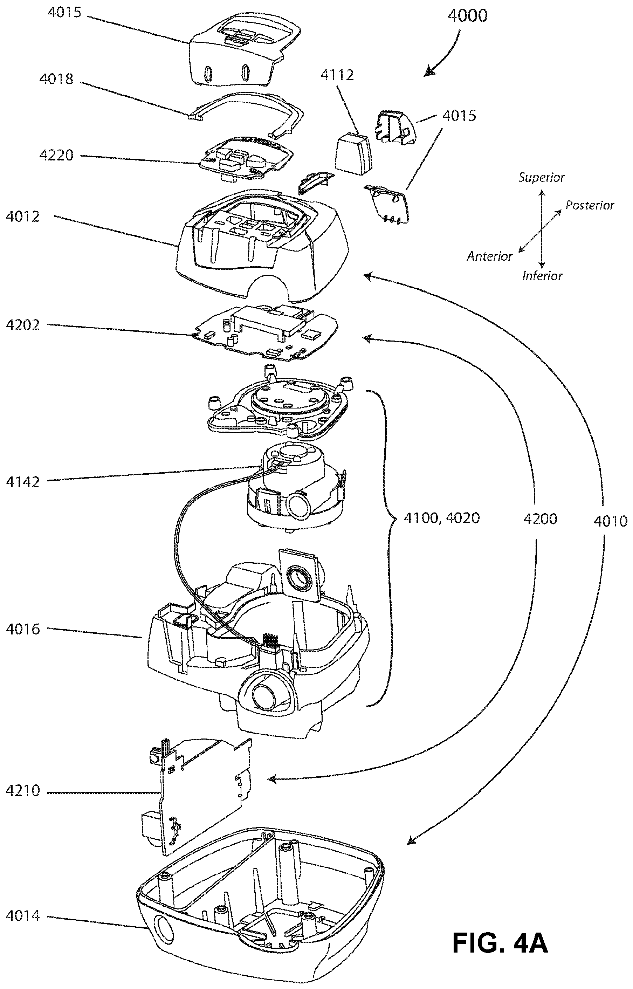

Fig. 4A shows an RPT device in accordance with one form of the present technology.

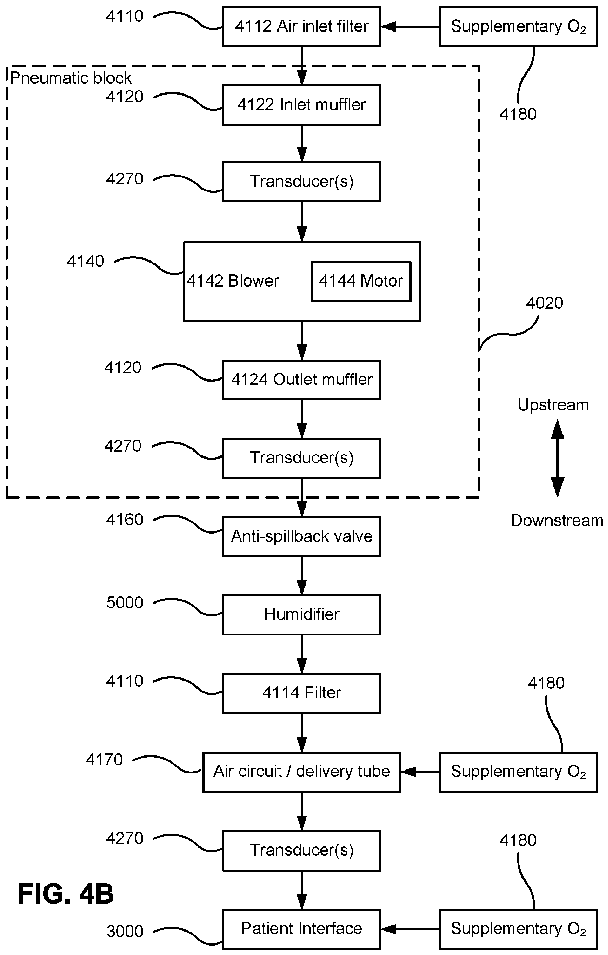

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

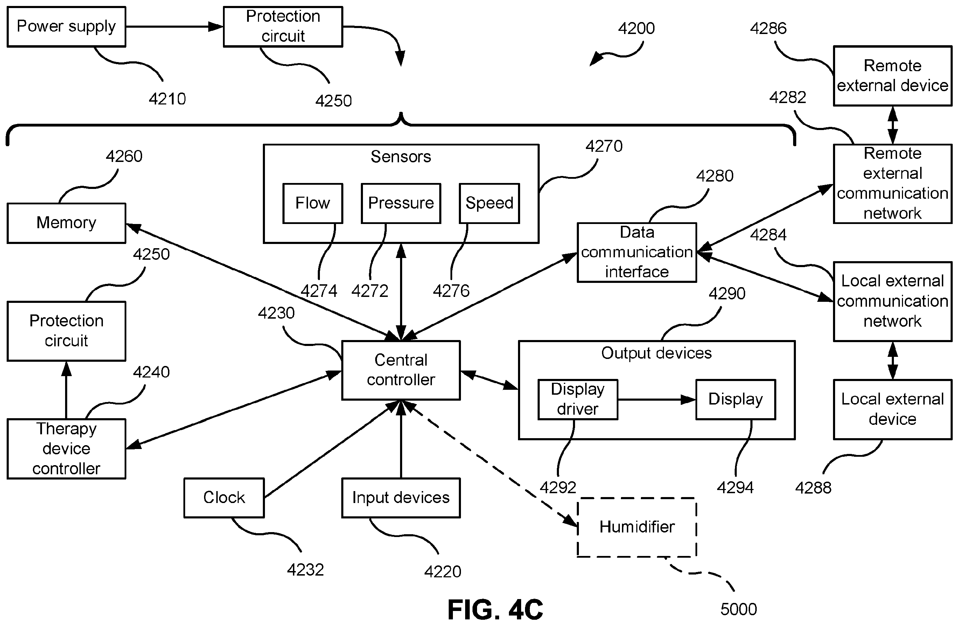

Fig. 4C is a schematic diagram of the electrical components of an RPT device in accordance with one form of the present technology.

### 4.4 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.
Fig. 5C shows a schematic of a humidifier in accordance with one form of the present technology.

### 4.5 CONNECTIVITY

Fig. 6A shows an RPT device in accordance with one form of the present technology.
Fig. 6B shows an end view of the RPT device of Fig. 6A
Fig. 6C shows a further end view of the RPT device of Figs. 6A and 6B.
Fig. 7A shows a connectivity module in accordance with one form of the present technology.
Fig. 7B shows an RPT device comprising a connectivity module in accordance with one form of the present technology.
Fig. 7C shows the RPT device of Fig. 7A with the connectivity module removed.
Fig. 7D shows the RPT device of Figs. 7A-7C without a connectivity module.
Fig. 8A shows an exploded view of a connectivity module in accordance with one form of the present technology.
Fig. 8B shows a further exploded view of a connectivity module in accordance with Fig. 8A.
Fig. 8C shows a partial cross-sectional view of the connectivity module of Figs. 8A and 8B.
Fig. 8D shows an assembled view of the connectivity module of Figs. 8A-8C.
Fig. 9 partial cross-sectional view of a further connectivity module according to another form of the present technology.
Fig. 10 shows part of a RPT device according to one form of the present technology.
Fig. 11A shows part of a RPT device according to one form of the present technology.
Fig. 11B shows part of a RPT device according to a further form of the present technology.
Fig. 11C shows part of a RPT device according to a further form of the present technology.
Fig. 11D shows part of a RPT device according to a further form of the present technology.
Fig. 11E shows part of a RPT device according to a further form of the present technology.
Fig. 12A shows a connector assembly according to one form of the present technology.
Fig. 12B shows a rear view of the connector assembly of Fig. 12A.
Fig. 12C shows a partial side view of the connector assembly of Figs. 12A and 12B.

### 4.6 SCREENING, DIAGNOSIS AND MONITORING SYSTEMS

Fig. 13 shows a patient undergoing polysomnography (PSG). The patient is sleeping in a supine sleeping position.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to + 150 L/min while maintaining a positive pressure of at least 6 cmH₂O, or at least 10cmH₂O, or at least 20 cmH₂O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH₂O to about 20 cmH₂O, or in other forms up to about 30 cmH₂O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the central controller 4230.

### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 5.4.2.3 Central controller

In one form of the present technology, the central controller 4230 is one or a plurality of processors suitable to control an RPT device 4000.

Suitable processors may include an x86 INTEL processor, a processor based on ARM^{®} Cortex^{®}-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 5.4.2.4 Clock

The RPT device 4000 may include a clock 4232 that is connected to the central controller 4230.

### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, the data communication interface 4280 may be provided on a connectivity module 7000 as described herein.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

### 5.4.2.9.1 Display driver

A display driver 4292 receives as an input the characters, symbols, or images intended for display on the display 4294, and converts them to commands that cause the display 4294 to display those characters, symbols, or images.

### 5.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases, there may be separate limbs of the circuit for inhalation and exhalation. In other cases, a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

### 5.6 HUMIDIFIER

### 5.6.1 Humidifier overview

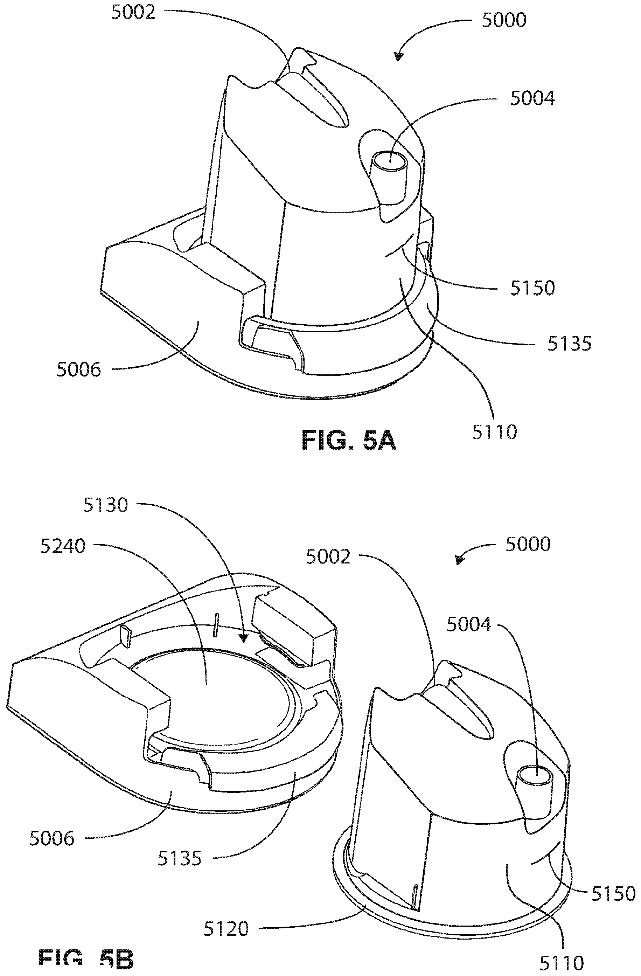

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, an inlet 5002 to receive a flow of air, and an outlet 5004 to deliver a humidified flow of *air.* In some forms, as shown in Fig. 5A and Fig. 5B, an inlet and an outlet of the humidifier reservoir 5110 may be the inlet 5002 and the outlet 5004 respectively. The humidifier 5000 may further comprise a humidifier base 5006, which may be adapted to receive the humidifier reservoir 5110 and comprise a heating element 5240.

### 5.6.2 Humidifier components

### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water. any portions thereof, such as 25%, 50% or 75% or volumes such as 200 ml, 300 ml or 400ml.

### 5.6.2.5 Humidifier transducer(s)

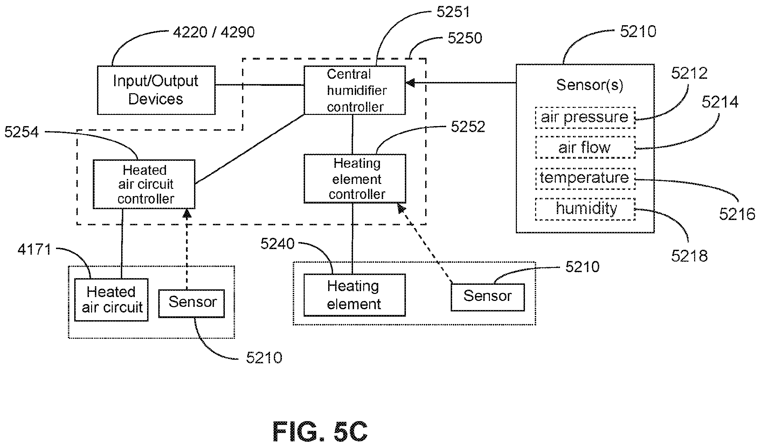

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 5C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

### 5.6.2.5.1 Pressure transducer

One or more pressure transducers 5212 may be provided to the humidifier 5000 in addition to, or instead of, a pressure sensor 4272 provided in the RPT device 4000.

### 5.6.2.5.2 Flow rate transducer

One or more flow rate transducers 5214 may be provided to the humidifier 5000 in addition to, or instead of, a flow rate sensor 4274 provided in the RPT device 4000.

### 5.6.2.5.3 Temperature transducer

The humidifier 5000 may comprise one or more temperature transducers 5216. The one or more temperature transducers 5216 may be configured to measure one or more temperatures such as of the heating element 5240 and/or of the flow of *air* downstream of the outlet 5004. In some forms, the humidifier 5000 may further comprise a temperature sensor 5216 to detect the temperature of the ambient air.

### 5.6.2.5.4 Humidity transducer

In one form, the humidifier 5000 may comprise one or more humidity sensors 5218 to detect a humidity of a gas, such as the ambient air. The humidity sensor 5218 may be placed towards the outlet 5004 in some forms to measure a humidity of the gas delivered from the humidifier 5000. The humidity sensor may be an absolute humidity sensor or a relative humidity sensor.

### 5.6.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO2012/171072.

In some forms, the heating element 5240 may be provided in the humidifier base 5006 where heat may be provided to the humidifier reservoir 5110 primarily by conduction as shown in Fig. 5B.

### 5.6.2.7 Humidifier controller

According to one arrangement of the present technology, a humidifier 5000 may comprise a humidifier controller 5250 as shown in Fig. 5C. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 5C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

### 5.7 CONNECTIVITY

### 5.7.1 Overview

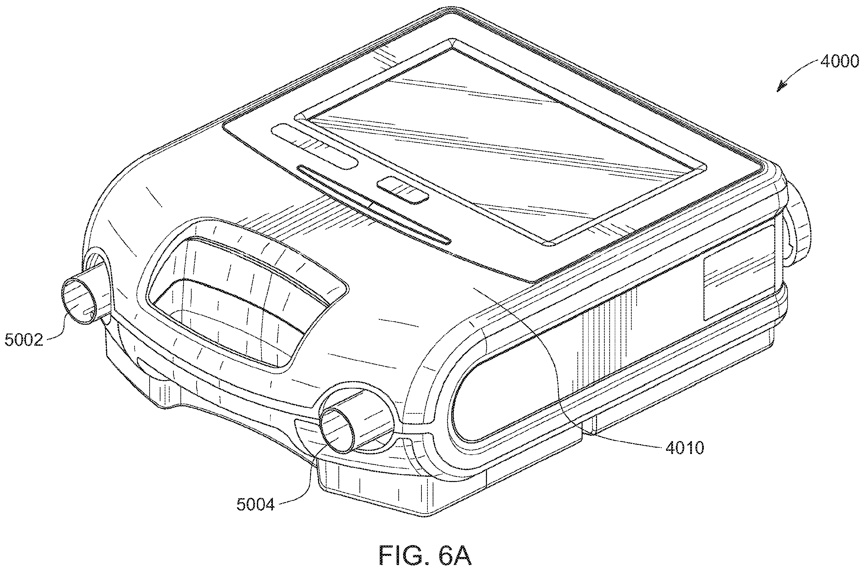

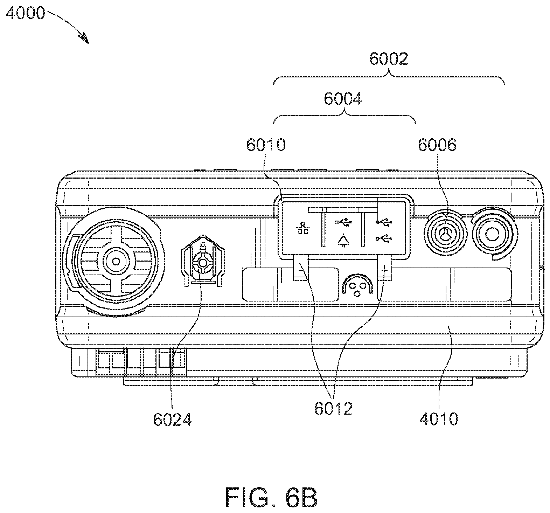

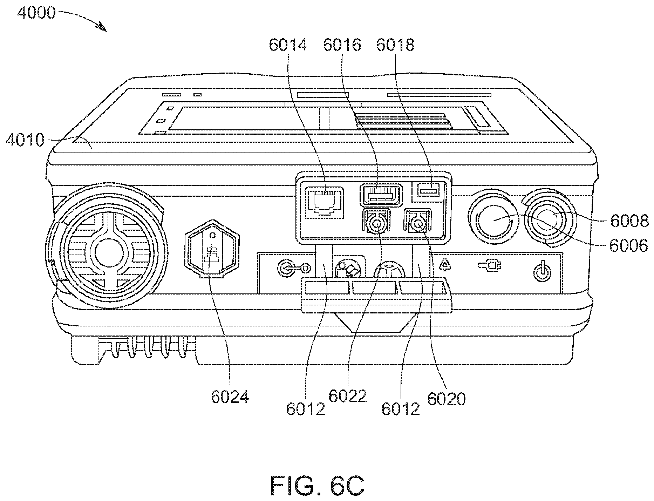

According to one form of the present technology, an RPT device 4000 is provided. In the example of Fig. 6A, 6B and 6C the RPT device 4000 includes an external housing 4010 which contains a pressure generator 4140 (not shown) for producing a flow of breathable gas at a positive pressure. For example, the pressure generator 4140 may be a controllable blower. The RPT device 4000 is provided with an inlet 5002 to receive a flow of air, and an outlet 5004 to deliver the flow of air to a patient for treatment of respiratory disorders via one or more respiratory therapies as described herein.

Figs. 6B and 6C show end views of the RPT device 4000 of Fig. 6A. Of particular note are the electrical connectors 6002 provided to the RPT device 4000.

The electrical connectors 6002 may comprise communication connectors 6004, which facilitate the transfer of information to or from the device, power connections 6006 which facilitate the transfer of power to or from the device, or any combination of these connectors including hybrid connectors which facilitate both power and data transfer. Also provided is a button 6008 which allows the device to be powered on and off.

In the example of Figs. 6A-6C, the RPT device 4000 includes an ethernet connector 6014, a USB-A connector 6016, a mini USB connector 6018, an SpO₂ sensor connector 6020 and a remote alarm connector 6022 which consists of five pins. Each of these connectors are arranged as a group which is optionally protected by a protective cover 6010. The power connection 6006, button 6008 and LFO₂ (low flow oxygen input) are positioned so as to not be covered by the protective cover 6010, however this should not be seen as limiting on the technology and in other forms these components may be covered by the protective cover 6010.

### 5.7.1.1 Communication Connectors

In forms of the technology where the RPT device 4000 includes communication connectors, it should be appreciated that these may consist of any suitable connector known in the art. For example, the communication connectors may comprise one or more electrical connector such as a D-subminiature connector (such as DB-9 or DE-9, etc.), a USB connector (such as USB type A, B or C connectors), an ethernet connector (such as an RJ-45 connector), or any suitable communication connector.

It should be appreciated that the communication connectors may facilitate the transfer of information to or from the RPT device 4000 using any suitable communications protocol. For example, information transfer may be achieved over RS-232, RS-485, SPI, I²C, USB, CAN bus, ethernet, or any other suitable communications protocol, including proprietary protocols.

The communication connectors may allow the RPT device 4000 to communicate with one or more peripheral device(s) such as a computer, data logging device, other RPT device 4000, humidifier 5000, smartphone or tablet. In some forms of the technology, the communication connectors provide a means to store and access data as required. For example, a USB connector may allow for files to be transferred to or from the device as required without requiring a connection to a computer, smartphone or tablet, i.e. the USB connector may allow for file transfer to or from a removable storage device.

The communication connectors may be used to facilitate the transfer of any forms of data. For example, the data may comprise software update data, usage information, such as information regarding use of the device, real-time information such as therapy pressure, humidity, leak information, forensic and/or diagnostic information (e.g. for troubleshooting the device or checking the health status of the device 4000), audio or visual data for purposes of providing information or alerts to the user via an external display or speaker. In addition, the communication connectors may facilitate the transfer of information during manufacturing or servicing the device 4000. For example data may be communicated during testing of the device and/or programming or configuring components of the device 4000.

Furthermore, while the foregoing examples are described as communication connectors, this should not be seen as limiting on the technology. For example, one or more of the communication connectors may additionally include power connections which provide power to or from the RPT device 4000. For example, the ethernet connection may comprise power-over-ethernet connections.

### 5.7.1.2 Power Connectors

In forms of the technology where the RPT device 4000 includes at least one power connector, it should be appreciated that this may consist of any suitable connector known in the art. For example, the power connectors may comprise one or more electrical connector such as a DC connector in the form of a barrel-jack, screw terminals, banana-plug connections or press-fit connector. Alternatively, or additionally, the RPT device 4000 may include AC power connectors such as IEC or NEMA standard power connectors (such as IEC 320 C13/C14/C7 or NEMA 5-15-P/1-15-P) as known to those in the art.

In forms of the technology, a power connection may be provided as part of a communication connector. For example, power may be provided via a USB or ethernet connection as should be understood by those skilled in the art.

### 5.7.1.3 Mechanical connectors

In forms of the technology, the RPT device 4000 may comprise one or more mechanical connectors configured to mechanically connect the RPT device 4000 to another component or system, such as air or oxygen connectors. In the example of Figs. 6A-6C a low flow oxygen connector (LFO₂) 6024 is provided.

### 5.7.1.4 Protective Cover

In Fig. 6B some of the electrical connectors 6002 are covered by a protective cover 6010 designed to protect the electrical connectors 6002 when they are not in use. For example, the protective cover 6010 may prevent or limit the ingress of dust, dirt or water into the electrical connectors 6002. By configuring the protective cover 6010 to cover one or more of a plurality of the electrical connectors 6002, it is possible for one or more electrical connectors 6002 to be in use while one or more unused connectors are covered by the protective cover. For example, one or more communication connectors may be covered by the protective cover 6010 while leaving one or more power connectors uncovered and available for use.

The protective cover 6010 shown in Fig. 6B is pivotally connected to the housing 4010 of the RPT device 4000 by at least one hinge 6012, for example two hinges as illustrated. The hinge(s) 6012 shown are formed of a resilient material such as an elastomer or polymer, however any suitable hinge 6012 construction may be used. In one form of the technology, the protective cover 6010 and/or hinge 6012 is formed during the moulding of at least part of the housing 4010 i.e. the protective cover 6010 or hinge 6012 is integral to or has a unitary construction with at least a part of the housing 4010.

In forms of the technology a protective cover 6010 may not be used and the electrical connectors 6002 may be exposed. In other forms of the technology, an alternative protective cover 6010 may be used. For example, the protective cover 6010 may be configured to clip, screw, press-fit or otherwise engage with the housing 4010 using alternative methods as should be known to those skilled in the art. In other forms of the technology, the protective cover 6010 may not engage with the housing 4010 but may be supported directly by the one or more of the electrical connectors 6002. For example, the protective cover 6010 may comprise one or more protrusions which engage with one or more of the electrical connectors 6002 so as to support the protective cover 6010.

### 5.7.2 Connector Assemblies

According to one or more forms of the present technology, it may be advantageous to provide one or more of the electrical connectors 6002 as part of a connector assembly 6030. For example, the electrical connectors 6002 may be attached to a common support member or section of the housing 4010, as will be described herein.

The connector assembly 6030 may comprise a plurality of electrical connectors 6002 which are mounted to or otherwise positioned in a common section of the housing 4010. For example, the electrical connectors 6002 may comprise panel mount connectors which are attached to the housing 4010 by passing through and engaging with apertures in the housing 4010. Alternatively, the panel mount connectors may comprise one or more fasteners such as nuts which can be tightened against the outer or inner faces of the housing 4010 in order to secure the connectors to the housing 4010. The connector assembly 6030 may therefore comprise a section of the housing 4010 together with one or more electrical connectors 6002.

The electrical connectors 6002 may also comprise PCB (printed circuit board) mounted connectors. In some forms of the technology, these PCB mounted connectors may be exposed through an aperture in the housing 4010 without directly contacting or engaging the housing 4010. In other forms of the technology, the PCB mounted connectors may pass through and engage with an aperture in the housing 4010. For example, the connectors may have a friction fit engagement with the housing. In other forms the connectors may include a sealing structure such as a flange, O-ring, or seal which engages with the housing 4010 in order to provide ingress protection. In yet further forms of the technology the housing 4010 may comprise sealing features such as areas of compliant materials (such as overmoulded polymers or elastomers) which engage with one or more of the electrical connectors 6002 in order to provide ingress protection. The connector assembly 6030 may therefore comprise a printed circuit board together with one or more electrical connectors 6002.

In yet further forms the electrical connectors 6002 may include cable mounted connectors. These cable mounted connectors may be attached to the housing 4010 by way of a grommet or other suitable mounting or strain relief features as should be known to those skilled in the art.

In yet further forms, one or more of the electrical connectors 6002 may be panel mount, PCB mount, and/or cable mounted.

It may be advantageous for the section of the housing 4010 which contains the one or more electrical connectors 6002 (i.e. connector assembly 6030) to be formed separately from the rest of the housing 4010 i.e. the connector assembly 6030 may comprise one or more electrical connectors together with a section of the housing 4010. It should be appreciated that, while the connector assembly 6030 may comprise a section of the housing 4010, this section of the housing may not be configured to be removed from the RPT device 4000 in normal use. In other words, an RPT device 4000 may comprise a connector assembly 6030 which is fixed to the housing in use.

There are a number of potential advantages to providing one or more electrical connectors in a connector assembly 6030. For example, advantages may include:
- Simplifying manufacture of the device; for example, connector apertures in the connector assembly 6030 may be moulded with a common line of draw to the moulding direction of the connector assembly 6030 i.e. the tooling required to produce the device may be less complex, reducing cost, improving moulding cycle times, improving part yield and moulding tool reliability.
- Allowing for streamlined assembly of the device; for example, the connector assembly 6030 may be assembled and tested independently of the rest of the RPT device 4000, thereby allowing for parallel manufacturing.
- Simplifying the servicing and replacement of the connectors 6030; for example, the RPT device 4000 may be disassembled and the connector assembly 6030 removed, replaced and the faulty unit sent for repair, thereby minimising the amount of time a unit needs to be under repair and reducing the level of expertise required of the service staff.
- Allowing for multiple product variants to be provided with a common device housing 4010; for example, different connector assemblies 6030 may be substituted during manufacturing in order to produce different product variants according to market requirements.
In one form of the technology the connector assembly 6030 may be fixed to the housing by engaging portions of the connector assembly 6030 with channels on the housing 4010. For example, when fixed, the edges of the connector assembly 6030 may be located in a recess or channel in the housing 4010 of the RPT device 4000 in use. In other forms of the technology, the connector assembly 6030 may be fastened to the RPT device 4000, for example by using one or more screws. In yet further forms of the technology, the connector assembly 6030 may be joined with the housing 4010, for example by using an adhesive or welding technique such as ultrasonic welding.

### 5.7.3 Connectivity Modules

In addition to the benefits of providing a connector assembly 6030 in an RPT device 4000, one form of the present technology relates to providing a modular, removable connector assembly herein referred to as a connectivity module 7000. It should be appreciated that throughout the present specification, reference to the connectivity module 7000 as removable should be understood to mean that it is able to be removed from the RPT device 4000 without disassembling or opening the RPT device 4000. The connectivity module 7000 may be provided in addition to the connector assembly 6030 or in place of the connector assembly 6030.

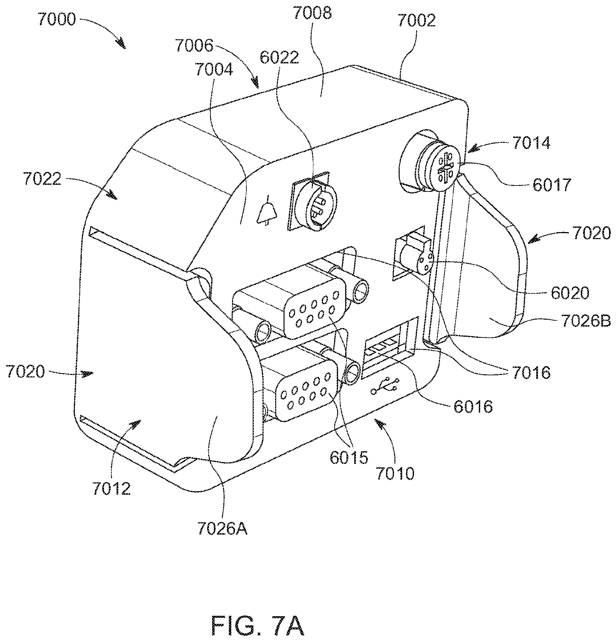

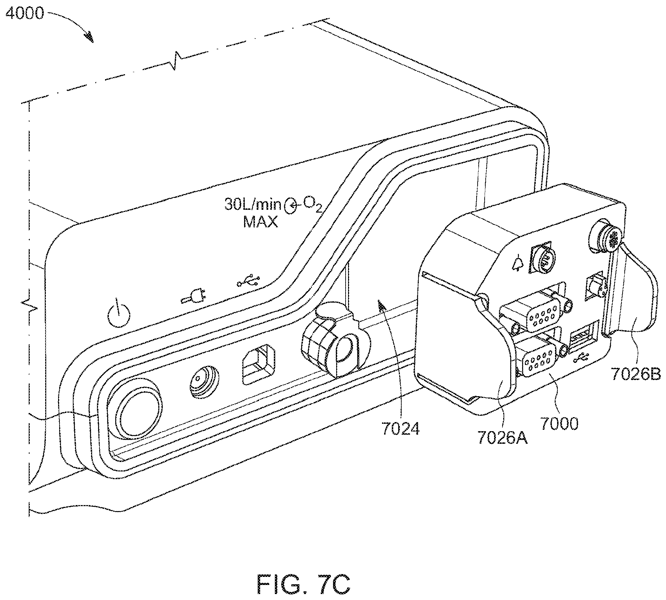

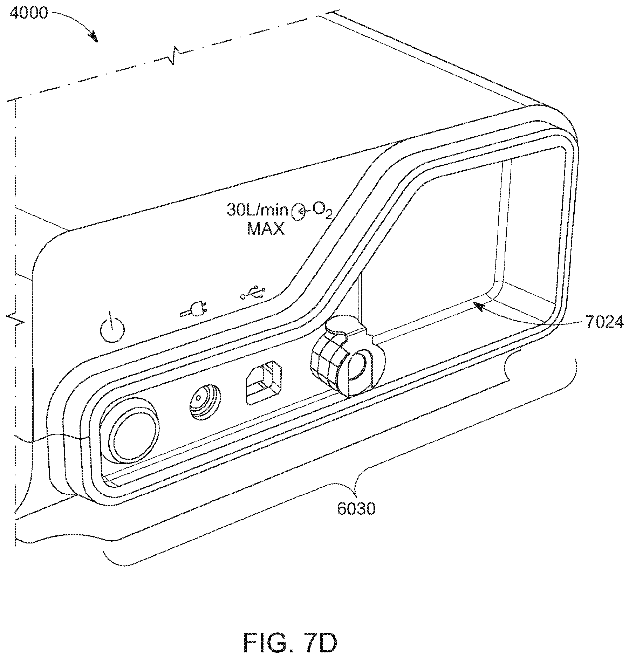

One form of a connectivity module 7000 according to the present technology is shown in Fig. 7A. In this form, the connectivity module 7000 comprises a housing 7002 which generally consists of a front surface 7004, a rear surface 7006, top surface 7008, bottom surface 7010 and sides 7012, 7014.

The front 7004 of the connectivity module comprises one or more electrical connectors 6002 that are attached to and/or extend through apertures 7016 in the front surface 7004.

The rear surface 7006 includes an interfacing connector 7018 (not shown) for electrically connecting the connectivity module 7000 to the RPT device 4000.

The sides 7012, 7014 of the housing 7002 comprise attachment mechanisms 7020, which are configured to in use secure the connectivity module 7000 to the RPT device 4000. In the illustrated form these attachment mechanisms 7020 are clips, however this should not be seen as limiting on the technology, and alternative attachment mechanisms 7020 are provided in other forms of the present technology. Furthermore, in other forms of the present technology the attachment mechanisms 7020 may be provided to another part of the housing 7002, for example the rear surface 7006, top surface 7008 and/or bottom surface 7010.

The connectivity module 7000 also includes one or more indexing features 7022, for example in the form of a chamfer. This indexing feature(s) 7022 ensures that the connectivity module is attached to the RPT device 4000 in the correct orientation as will be described in greater detail herein.

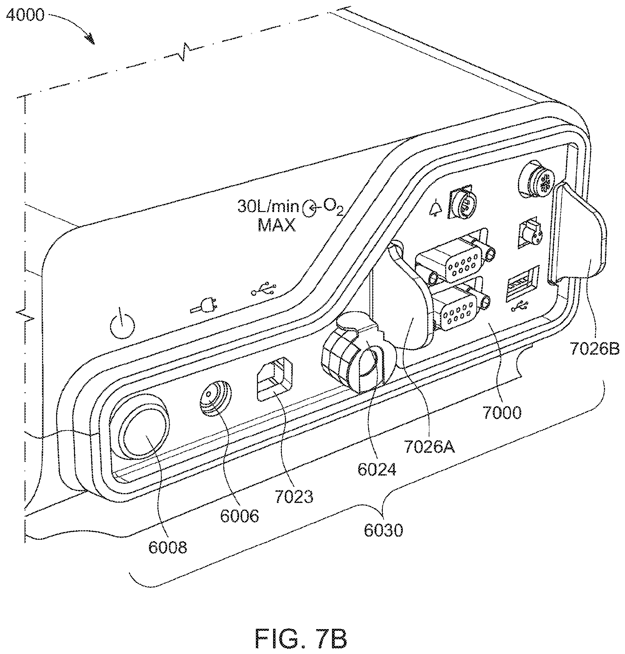

Figs. 7B-7D show examples of the connectivity module 7000 in various stages of connection with the RPT device 4000. In the form shown the RPT device 4000 comprises a cavity 7024 which is configured to receive the connectivity module 7000. Cavity 7024 is a space or recess in an outer surface of the RPT device 4000 into which the connectivity module 7000, or a part thereof, may be positioned. The cavity 7024 may be formed by a change in shape or direction of the outer surface of the RPT device 4000, for example a recessing of an outer surface or one or more gaps in an outer surface. The cavity 7024 shown in Figs. 7B-7D is provided in the rear of the RPT device 4000 (i.e. a surface on the opposite side from the user-facing side of the RPT device 4000, for example the side on which are located user controls), however this should not be seen as limiting on the technology, and in other forms, the cavity may be in a top, bottom, front or side surface of the RPT device 4000.

In the illustrated form, the cavity 7024 is provided in a connector assembly 6030 comprising at least one electrical connector 6002. However, this should not be seen as limiting on the technology, and in other forms, the connectivity module 7000 may be configured to engage with a cavity 7024 in the housing directly, or otherwise attach to the RPT device 4000 without engaging with or being retained within a cavity 7024.

Positioning the connectivity module 7000 within a cavity 7024 in the connector assembly 6030 or housing 4010 may advantageously:
- Provide a more secure connection between the connectivity module 7000 and RPT device 4000. For example, positioning the connectivity module 7000 within the cavity 7024 may provide greater protection against accidental disconnections due to being knocked or bumped, particularly during transportation.
- Provide a measure of strain relief on the interfacing connector(s) 7018 by reducing or limiting the forces imparted to the interfacing connector 7018 as the RPT device 4000 or the cables connecting to the electrical connectors 6002 are moved or twisted.
- Simplify alignment of the interfacing connector 7018 between the connectivity module 7000 and RPT device 4000 as the complementary shapes of the connectivity module and housing can guide the interfacing connectors 7018 into alignment.

- Provide greater ingress protection by recessing the interfacing connectors 7018 within the cavity 7024.
- Provide the RPT device 4000 with a sleek appealing aesthetic.

In forms of the technology, the connectivity module 7000 may be positioned entirely within the cavity 7024 in its connected configuration. For example, the connectivity module 7000 may be configured so that, when received in the cavity 7024, the front surface 7004 of the connectivity module 7000 may be substantially aligned with, or may be recessed from, the outer surface of the housing 4010 or connector assembly 6030.

In other forms of the technology, the connectivity module 7000 may be partially positioned within the cavity 7024. For example, the connectivity module 7000 may extend at least partially outwardly of the cavity 7024. For example, the connectivity module 7000 may extend outwardly of the cavity 7024 between approximately 5mm and approximately 25mm.

In other forms of the technology, the connectivity module 7000 may connect directly to the connector assembly 6030 or housing 4010 of the RPT device 4000 without being located in a cavity.

In some forms of the technology, the cavity 7024 may be angled downwardly relative to the RPT device 4000. For example, when the RPT device is positioned on a horizontal surface, the cavity may be angled downwardly relative to the horizontal surface. Angling the cavity downwardly may advantageously facilitate the draining of fluids from the cavity, thereby improving the ingress protection of the RPT device 4000.

As illustrated in Figs. 7A-7D the connectivity module comprises at least one electrical connector 6002. In the form shown, the connectivity module comprises two 9-pin D-subminiature connectors 6015. a female USB-A connector 6016, an SpO₂ (peripheral capillary oxygen saturation) sensor connector 6020 a FiO₂ (fraction of inspired oxygen) sensor connector 6017 and a remote alarm connector 6022. However, this should not be seen as limiting on the technology, and any number and arrangement of connectors may be used in accordance with the present technology.

In addition to the connectors provided on the connectivity module 7000, the connector assembly 6030 includes a power button 6008, DC power connector 6006, female USB-B connector 7023, and LFO₂ (low flow oxygen) connector 6024. Advantages of providing separate groups of connectors on the removable connectivity module 7000 and non-removable connector assembly 6030 include providing:
- A base or core set of connectors on the connector assembly 6030 which are present across multiple product variants, i.e. a power connection 6006 and power button 6008.
- Connectors which are non-essential to all customers or product variants on the connectivity module 7000; for example, remote alarm monitoring, FiO₂ or SpO₂ sensor connections.
- Mechanical or pneumatic connections such as LFO₂ directly on the connector assembly 6030, in order to simplify the interfacing connection 7018 between the connectivity module 7000 and RPT device 4000 (i.e. no pneumatic connection required).

### 5.7.3.1 Attachment Mechanisms

In forms of the technology comprising a removable connectivity module 7000, one or more attachment mechanisms 7020 may be provided to secure the connectivity module 7000 to the housing 4010, cavity 7024 or connector assembly 6030 of the device 4000. For sake of simplifying the foregoing discussion the foregoing examples describe mechanisms for attaching the connectivity module 7000 at least partially within a cavity 7024. This however should not be seen as limiting on the technology, and it should be appreciated that the foregoing attachment mechanisms may be used to attach a connectivity module to any component of an RPT device 4000.

### 5.7.3.1.1 Clips

According to one form of the present technology the attachment mechanism 7020 may comprise one or more clips 7026A, 7026B as illustrated in Figs. 7A-7D. In the form shown, a first clip 7026A is provided on the first side 7012 of the connectivity module 7000 and a second clip 7026B is provided on the second side 7014 of the connectivity module 7000. First clip 7026A and second clip 7026B are structured and arranged to clip into suitable clip receiving portions on the RPT device 4000. The clips may be snap-fit connectors, press-fit connectors or any suitable form of clip connector.

In the illustrated examples, the clips 7026A, 7026B extend outwardly of the front surface 7004 of the connectivity module 7000 so as to provide an extension which a user may engage in use. For example, the user may squeeze the clips 7026A, 7026B inwardly towards each other to release the connectivity module 7000 from the RPT device 4000. The clips 7026A, 7026B may further comprise a gripping portion or textured surface to further facilitate engagement by a user. However, this should not be seen as limiting on the technology, and in other forms of the technology, the clips 7026A, 7026B may not include gripping portions or extend outwardly of the connectivity module 7000. For example, the clips may extend along one or more sides of the connectivity module 7000 without extending beyond the front surface 7004 of the connectivity module 7000. In other forms of the technology, the clips may be provided on part of the side walls of the connectivity module. In some forms of the technology it may be advantageous to provide a tool to facilitate removal of the connectivity module 7000 from the RPT device. For example, a shim may be passed along the sides of the connectivity module 7000 to release the clips 7026A, 7026B from the cavity 7024. Requiring a tool for removal of the connectivity module 7000 may advantageously prevent tampering of the device in a public setting, such as in a hospital, sleep lab, or medical clinic.

In one form of the technology, the clips 7026A, 7026B may include one or more protrusions, indentations or apertures (not shown) on the sides of the clips, which are configured to engage with corresponding protrusions, indentations or apertures on the internal walls of the cavity 7024 (not shown). In other forms of the technology the clips may engage with the internal walls of the aperture by way of an interference fit or friction fit.

The clips 7026A, 7026B may be resiliently attached to the connectivity module 7000 such that insertion of the connectivity module 7000 into the cavity 7024 biases the clips inwardly towards the connectivity module 7000. This biasing may improve the engagement between the clips 7026A, 7026B and the internal walls of the cavity 7024 or the protrusions, indentations and/or apertures thereof.

In use, to release the connectivity module 7000 from the cavity 7024, a user may squeeze the first clip 7026A and second clip 7026B inwardly towards each other to therefore reduce the engagement force between the connectivity module 7000 and cavity 7024 or protrusions, indentations and/or apertures thereof.

It should be appreciated that while the clips 7026A, 7026B are shown on the sides of the connectivity module, in other forms of the technology, clips may instead be provided on the top 7008 and/or bottom 7010 surfaces of the connectivity module 7000. In yet further forms the clips may be provided on the RPT device 4000 for example, the clips may be provided on connector assembly 6030 or the inner walls of the cavity 7024 instead of the connectivity module 7000. In yet further forms of the technology, a clip 7026A, 7026B may be provided on a single side 7012, 7014 of the connectivity module 7000, and the opposing side 7012, 7014 of the connectivity module 7000 may be secured to the cavity 7024 using any of the other attachment mechanism 7020 described herein.

### 5.7.3.1.2 Fasteners

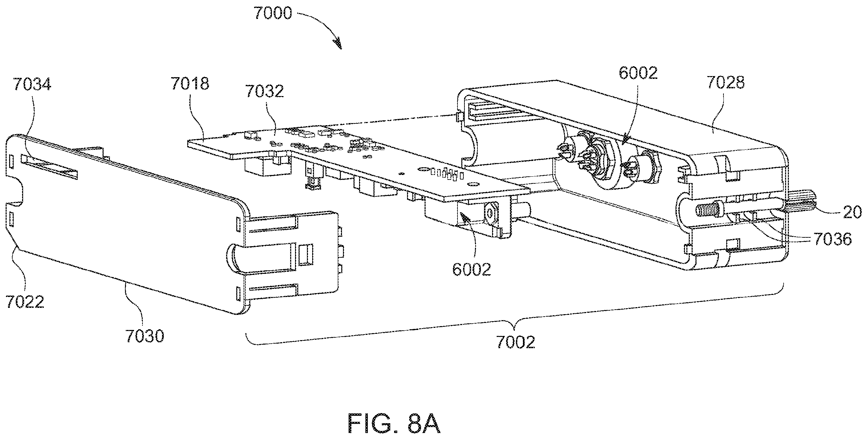

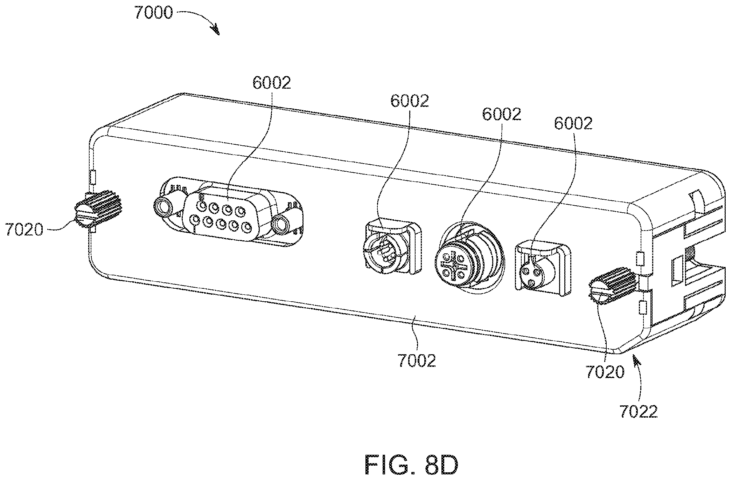

According to one form of the present technology, the connectivity module 7000 may be releasably attached to the cavity 7024 using one or more fasteners. For example, at least one screw or bolt may be used to secure the connectivity module 7000 to the cavity 7024 as will be described in greater detail in relation to Figs. 8A-8D and 8E.

In one form, the connectivity module 7000 comprises a screw or bolt, and the cavity 7024 comprises a thread configured to receive the screw or bolt. For example, the screw may be a thumb screw and the thread may be provided by a captive nut, threaded insert or threaded aperture. In other forms of the technology, the cavity 7024 may comprise a screw or bolt which extends through an aperture in the connectivity module 7000. In this form, securing the connectivity module 7000 to the cavity 7024 may be achieved by engaging a nut or other threaded member with the screw or bolt in order to fasten the connectivity module 7000 to the cavity 7024.

In one form of the technology, the fasteners may comprise thumb screws, wingnuts or other fasteners designed to be actuated without the use of tools. This may advantageously simplify the process of securing and or removing the connectivity module 7000.

In yet further forms the fasteners may include one or more security fasteners such as a tamper proof screws sold under the Torx registered trademark. Use of security fasteners may advantageously prevent tampering of the device in a public setting, such as in a hospital, sleep lab, or medical clinic.

### 5.7.3.1.3 Apertures and Protrusions

According to one form of the present technology the connectivity module 7000 may attach to the cavity 7024, by way of engagement of one or more apertures or indentations with one or more protrusions. In some forms this may be a clip as described herein, i.e. the protrusion may "clip" into the aperture. However, in other forms the protrusion may be a substantially rigid member which is inserted into an aperture in use.

For example, a protrusion may be provided on a first side of the connectivity module 7000 which is configured to, in use engage with an aperture on an internal wall of the cavity 7024. In other examples, a cavity may be provided on a first side of the connectivity module 7000 which is configured to, in use engage with a protrusion on an internal wall of the cavity 7024.

In some forms of the technology, the connectivity module may be configured so that, in order to connect it to the RPT device 4000, a user inserts a protrusion on a first side 7012 of the connectivity module 7000 into an aperture on an internal wall of the cavity 7024 in advance of inserting the second side 7014 of the connectivity module 7000 into the cavity 7024. For example, a connectivity module 7000 may comprise a protrusion on a first side 7012 and a clip on the second side 7014 (or vice versa). In use, a user may insert the protrusion into an aperture on a first side of the cavity 7024, then insert the second side 7014 of the connectivity module 7000 into the aperture 7024 in order to engage the clip with a corresponding indentation or aperture in the second side of the cavity 7024.

### 5.7.3.1.4 Interfacing Connector

According to a further form of the present technology, the connectivity module 7000 may attach to the cavity 7024 by way of the interfacing connector 7018. For example, retention of the connectivity module 7000 within the cavity 7024 may be provided by a locking or retention feature on the interfacing connector 7018. Additionally, or alternatively, the connectivity module 7000 may be retained in the cavity by the friction provided by the interfacing connector 7018.

### 5.7.3.1.5 Magnetic Attraction

According to a further form of the present technology, the RPT device 4000 and connectivity module 7000 may be provided with complementary magnetic members (not shown). Reference to magnetic members throughout the present specification should be understood to include ferromagnetic materials capable of magnetic attraction, as well as permanent and temporary magnets (including electromagnets).

For example, the connectivity module 7000 may be provided with a first magnetic member, and the RPT device 4000 may be provided with a second magnetic member. For example, the first magnetic member may be formed of a magnetic material such as steel or iron, while the second magnetic member may comprise a permanent magnet (such as a neodymium or ceramic magnet), or vice versa.

In one form of the technology, the connectivity module 7000 is provided with a first magnetic member proximate to a first side 7012 of the connectivity module 7000, and a second magnetic member proximate to a second side 7014 of the connectivity module 7000. The first magnetic member and second magnetic member may be positioned adjacent to the rear 7006 of the connectivity module. In this example, the RPT device 4000 comprises a third magnetic member and fourth magnetic member, each positioned within or proximate to the cavity 7024 such that upon insertion of the connectivity module 7000 into the cavity 7024, the first magnetic member is attracted towards the third magnetic member, and the second magnetic member is attracted towards the fourth magnetic member, so as to retain the connectivity module 7000 at least partially within the cavity.

The use of magnetic attraction to retain the connectivity module 7000 within the cavity 7024 may have a number of advantages including:
- Allowing for a compact unobtrusive attachment mechanism, particularly as the magnetic attachment mechanisms may be hidden within the housing 4010 of the RPT device 40000 and connectivity modules 7000 respectively.
- Providing improved ingress protection as the attachment mechanism 7020 can be sealed entirely within the housing 4010 of the RPT device 40000 and connectivity modules 7000 respectively.
- Providing a fast, tool-less installation with haptic feedback to the user to indicate correct attachment.
- Improved engagement between the interfacing connectors 7018.

### 5.7.3.1.6 Feedback Mechanisms

In forms of the technology, the attachment mechanisms may be configured to provide positive audible, tactile or haptic feedback to the user that the connectivity module 7000 has been correctly inserted. This may be provided by the attachment mechanisms 7020 themselves, or otherwise be provided by a feedback mechanism as should be known to those in the art.

For example, where magnetic attraction is used, it may be possible for the magnetic attraction to be significantly strong to rapidly pull the connectivity module 7000 into engagement with the RPT device 4000. This rapid engagement may provide an audible and/or haptic/tactile click as the connectivity module 7000 contacts the RPT device 4000.

In other forms of the technology, connection of the interfacing connectors 7018 may provide audible, haptic or tactile feedback to the user.

In yet further forms of the technology, a tactile mechanism may be provided. For example, the inner walls of the cavity 7024 may comprise indentations or protrusions which are configured to interface to with corresponding indentations or protrusions in the outer surface(s) of the connectivity module 7000. During insertion of the connectivity module 7000 into the cavity 7024, engagement of the indentations and protrusions may provide audible, haptic or tactile feedback to the user that the connectivity module has been inserted correctly.

In yet further form of the technology, feedback may be provided to the user by one or more audio or visual alerts from the RPT device 4000. For example, the RPT device may be configured to detect the connection of the connectivity module 7000 and generate a visual alert using a display or light, or an audible alert using a buzzer or speaker.

### 5.7.3.1.7 Combinations

It should be understood that the aforementioned attachment mechanisms are not exclusive of one another and, in forms of the technology, one or more of the attachment mechanisms may be combined to attach the connectivity module 7000 to the RPT device 4000. For example, the RPT device 4000 and connectivity module 7000 may include a combination of any one of clips, fasteners, apertures and protrusions, interfacing connectors, magnets, and/or feedback mechanisms.

### 5.7.3.2 Structure

### 5.7.3.2.1 Housing

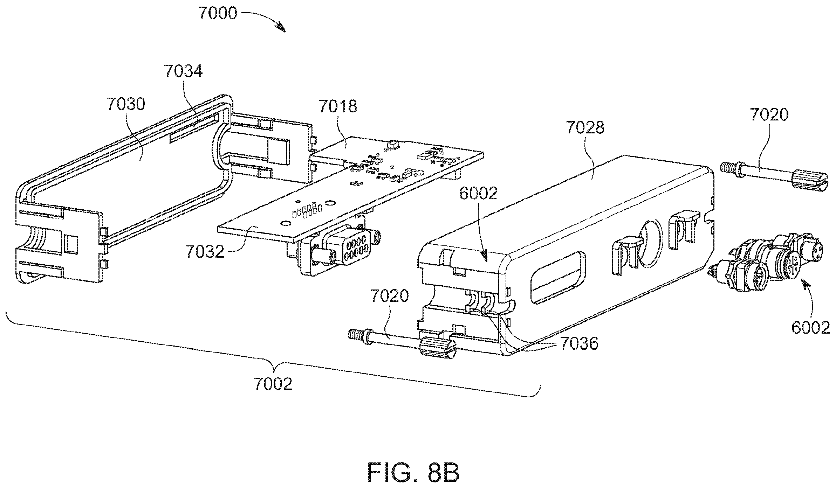

According to one form of the technology, the connectivity module 7000 comprises a multi-part housing 7002 as illustrated in Figs. 8A and 8B. In this example, the housing has a two-part construction consisting of a shell 7028 and backplate 7030. However, this should not be seen as limiting on the technology and in other forms the housing 7002 may be formed in other ways. For example, in certain forms, the housing 7002 may also comprise a two-part construction but a side surface of the housing 7002 other than the backplate 7030 may be a part that is removable from the rest of the housing 7002, for example the housing 7002 may comprise a removable front plate, side plate or top plate. In other forms, the housing 7002 may comprise any number of separable components, for example three, four or more components.

In use the shell 7028 and backplate 7030 are joined together in order to provide a compartment which contains the connectors and PCB 7032. The shell 7028 and backplate 7030 may be joined together using any methods known in the art, such as fasteners, clips or adhesives.

According to one form of the technology, it may be desirable for the connectivity module 7000 to be substantially sealed against water and dust ingress. One method of achieving this is to provide a sealant on or around the mating surfaces of the shell 7028 and backplate 7030. In other forms, a gasket or compression seal may be provided between the shell and backplate which is compressed in use to provide the seal. In further forms of the technology, the shell 7028 may be bonded to the backplate 7030, for example by ultrasonic welding.

In further forms of the technology, it may be desirable to protect the electrical connectors 6002 and PCB 7032 directly. For example, the PCB 7032 and/or connectors may be protected using a resilient water-proof coating, such as a conformal coating or polyurethane or epoxy resin. It should be appreciated that this may be used in place of or in addition to the options previously discussed for sealing the housing.

The connectivity module 7000 also comprises attachment mechanisms 7020 in the form of fasteners. The fasteners are rotatably attached to the first side 7012 and second side 7014 of the housing 7002 such that they do not pass through the sealed compartment which contains the electrical connectors 6002. For example, the housing 7002 may comprise one or more ribs 7036 on an outer surface. These ribs may be configured to receive and/or retain the fasteners 7020. This arrangement may simplify the sealing of the connectivity module 7000 as there is no need to seal around any moving parts.

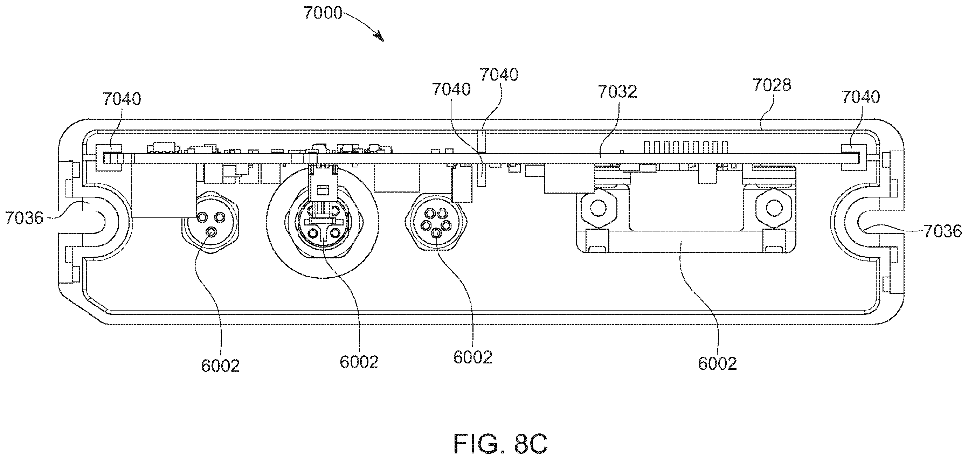

As shown in Fig. 8C, the housing 7002 may comprise one or more mounting features 7040 designed to support the PCB 7032 in use. For example, in the form shown the mounting features 7040 are ribs or extrusions formed in the shell 7028 which provide a channel in which the PCB 7032 can sit.

### 5.7.3.2.2 Electronics

In the illustrated example of Figs. 8A-8D the electrical connectors 6002 comprise a combination of panel mount connectors which are shown attached to the shell, and PCB mount connectors which are mounted to the PCB 7032. However, this should in no way be seen as limiting on the scope of the technology.

Also apparent in this example is a slot 7034 in the rear wall 7006 or backplate 7030 of the housing 7002 which is configured to accommodate the interfacing connector 7018. In the illustrated form, the interfacing connector 7018 comprises edge contacts (otherwise known as gold fingers). However, this should in no way be seen as limiting on the technology, and any suitable connector may be used, including ribbon cable connectors. In a yet further form of the technology the PCB 7032 may comprise one or more flexible PCB layers, and the interfacing connector 7018 may be provided by extending one or more of the flexible PCB layers through the slot in the rear surface 7006.

In forms of the technology, the PCB 7032 may comprise electronic components. For example, the PCB 7032 may comprise one or more of:
- Protection components, such as transient voltage suppressors;
- Filtering components such as inductors, capacitors and resistors;
- Communication components, such as biasing resistors to hold the communication lines in a known state;
- Isolation components, such as optical or inductive isolators;
- Processors, such as microcontrollers or microprocessors;
- Output devices such as LEDs, speakers or buzzers, for example a status LED may be provided on the connectivity module in order to indicate that it has been attached correctly; and
- Identification components, for example, the RPT device 4000 may communicate with the connectivity module 7000 in order to determine the type of connectivity module 7000 connected (i.e. what connections have been provided).

In a yet further form of the technology, the connectivity module 7000 may be provided with components which enable the connectivity module 7000 to communicate with, receive power from, or provide power to the RPT device 4000 without use of an interfacing connector 7018. For example, the PCB 7032 may comprise components that enable short range wireless communication or power transfer. For example, in one form the connectivity module 7000 comprises a wireless power transfer module configured to transfer power to/from the RPT device 4000 wirelessly, such as using inductive coupling. Alternatively or additionally, the connectivity module 7000 may comprise one or more wireless communication devices configured to transfer data to/from the RPT device 4000, such as a Bluetooth or Wifi module. Use of wireless communication and/or power transfer may advantageously allow for easier sealing of the connectivity module 7000.

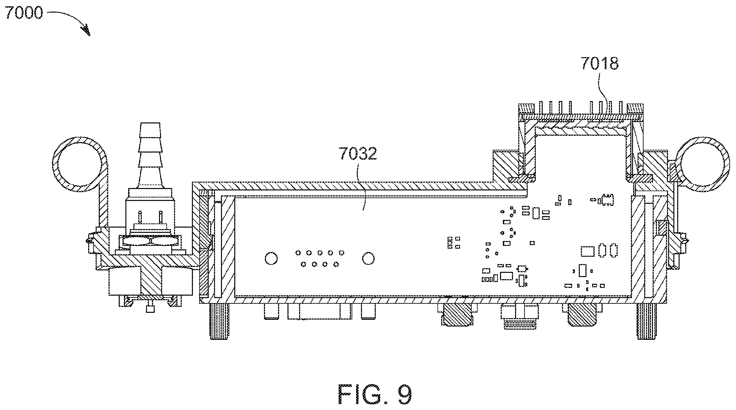

Fig. 9 shows a further cross-sectional view of a connectivity module 7000 according to another form of present technology. In this example, the interfacing connector 7018 is an electronic connector which comprises a first mating half located on the PCB 7032 and a second, complementary, mating half located on the RPT device 4000.

### 5.7.3.2.3 Exemplary shapes / dimensions

The connectivity module 7000 illustrated in Figs. 8A-8D has a substantially regular shape, such as a rectangular cuboid, however this should not be seen as limiting on the technology. In other forms of the technology, the connectivity module 7000 may have an irregular shape such as an "L" shape. The use of irregular shapes may advantageously ensure that the connectivity module 7000 is inserted into the cavity 7024 in the correct orientation without requiring the use of another specific indexing feature 7022 (as the shape of the connectivity module 7000 itself then acts as the indexing feature).

In one form of the technology, the connectivity module 7000 has:
- A thickness of between approximately 15mm and approximately 35mm for example between approximately 20mm and approximately 30mm, or more preferably approximately 25mm.
- A height of between approximately 50mm and approximately 80mm for example between approximately 60mm and approximately 70mm, or more preferably approximately 65mm.
- A width of between approximately 75mm and approximately 100mm for example between approximately 85mm and approximately 95mm, or more preferably approximately 90mm.

It should be appreciated that the aforementioned dimensions are provided by way of example only, and exclude the dimensions of any attachment mechanisms 7020 or interfacing connectors 7018.

### 5.7.4 RPT Device

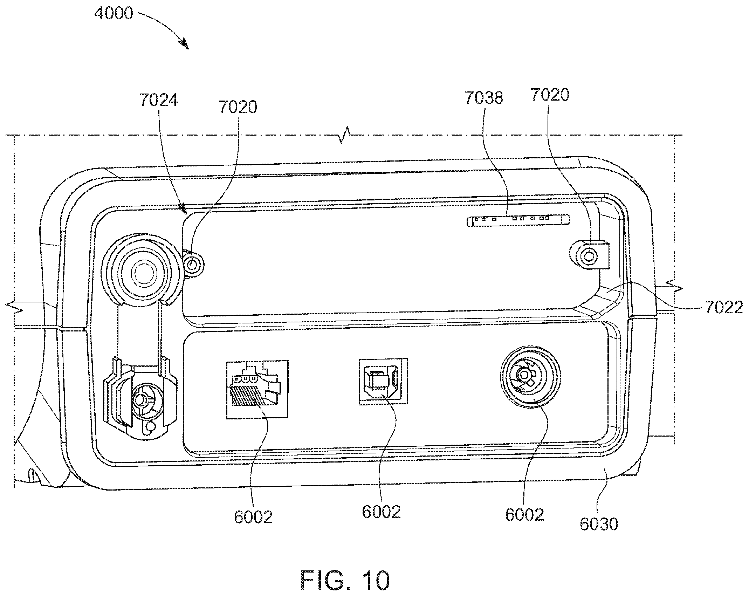

Fig. 10 shows a rear view of an RPT device 4000 according to the present technology. As shown, the RPT device 4000 includes a connector assembly 6030 which comprises one or more electrical connectors 6002, and a cavity 7024 configured to receive a connectivity module 7000 in accordance with the present technology.

The RPT device 4000 includes attachment mechanisms 7020 in the form of threaded inserts which are configured to receive a threaded fastener from a connectivity module 7000 in use.

The cavity 7024 includes a slot 7038 configured to receive an interfacing connector 7018 in use. It should be appreciated that in use, the interfacing connector 7018 is inserted through the slot 7038 in use in order to electrically connect to a corresponding connector within the RPT device 4000. In other forms the cavity may comprise a connector which the interfacing connector 7018 attaches to in use. In other words, in some forms the interfacing connector 7018 may not pass through a slot 7038.

The cavity 7024 has a complementary shape to the connectivity module 7000. For example, in one form of the technology, the cavity 7024 may have:
- A depth of between approximately 15mm and approximately 35mm for example between approximately 20mm and approximately 30mm, or more preferably approximately 25mm.
- A height of between approximately 50mm and approximately 80mm for example between approximately 60mm and approximately 70mm, or more preferably approximately 65mm.
- A width of between approximately 75mm and approximately 100mm for example between approximately 85mm and approximately 95mm, or more preferably approximately 90mm.

### 5.7.4.1 Exemplary Devices

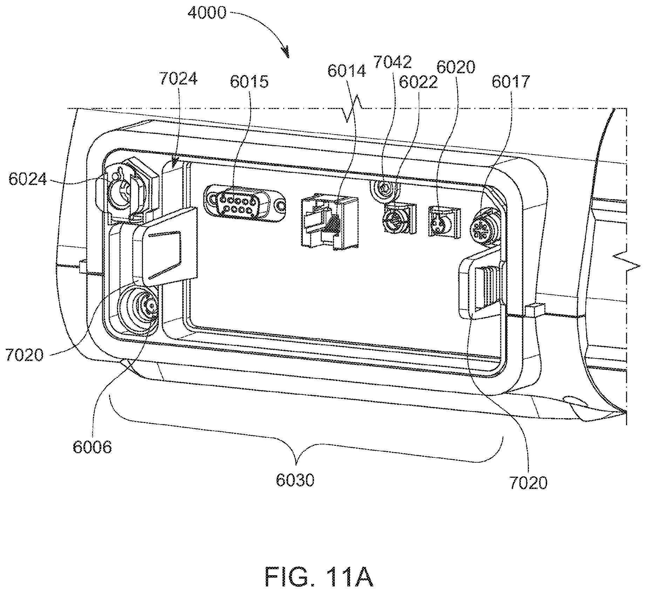

Figs. 11A-11E show various forms RPT devices 4000 comprising connectivity modules 7000 according to the present technology.

Fig. 11A shows one form of a connector assembly 6030 comprising a connectivity module 7000 according to the present technology. In this form the connectivity module 7000 is positioned within a cavity 7024 in the connector assembly 6030. Attachment mechanisms 7020 are provided in the form of clips.

A fastener 7042 is also provided which may be used to secure the shell 7028 and backplate 7030 of the housing 7002 together. In other forms, the fastener 7042 may be used in combination with the clips to secure the connectivity module to the cavity 7024.

In the example shown in Fig. 11A the connector assembly is primarily occupied by the connectivity module 7000. That is to say that the connectivity module takes between approximately 70% and 90% of the visible area of the connector assembly 6030. This may advantageously provide room for additional connectors to be provided on alternative connectivity modules 7000.

In the exemplary form of the technology shown in Fig 11A. the connectivity module 7000 comprises a D-subminiature connector 6015, an ethernet connector 6014, remote alarm connection 6022, SpO₂ sensor connection 6020, and an FiO₂ sensor connection 6017. The connector assembly 6030 also includes a LFO₂ connector 6024 and a DC power connector 6006.

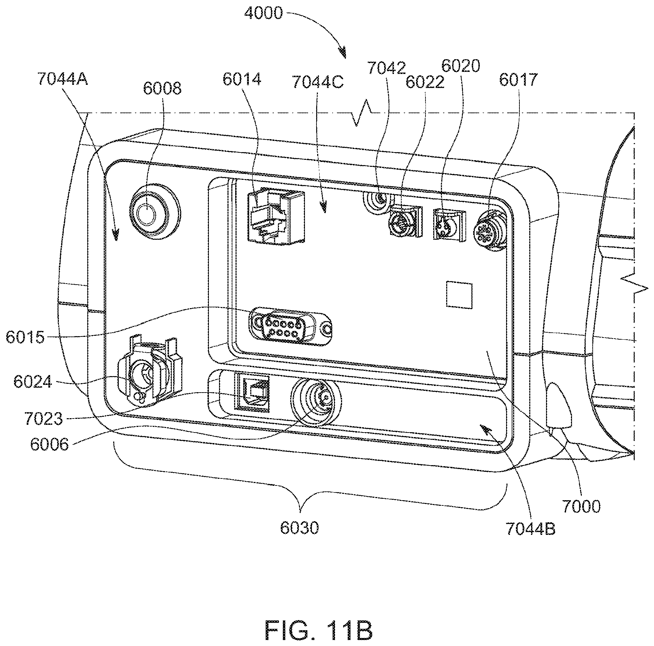

Fig. 11B illustrates a further form of the present technology, wherein the attachment mechanism 7020 is provided by way of a fastener 7042 (not shown). In this example the connector assembly 6030 is divided into three regions. The first region 7044A, has a power button 6008 and LFO₂ connector 6024. The second region 7044B includes electronic connectors which may be present on many RPT devices 4000. In the illustrated example these include a DC power connection 6006 and USB-B connection 7023. The third region 7044C provides the cavity 7024 for receiving the connectivity module 7000, in accordance with the present technology. In the illustrated exemplary form, the connectivity module 7000 comprises a D-subminiature connector 6015, an ethernet connector 6014, remote alarm connection 6022, SpO₂ sensor connection 6020, and an FiO₂ sensor connection 6017.

In certain forms of the present technology, more than one of the regions 7044A-C may be configured to connect to a connectivity module 7000. For example, in the example of Fig. 11B the connectors provided in the second region 7044B are part of the connector assembly 6030, however in alternative forms of the technology, these connectors may be comprised as part of a further connectivity module 7000 which is distinct from the connectivity module 7000 that connects to the third region 7044C. In other words, the RPT device 4000 may comprise more than one connectivity module 7000 in accordance with the present technology.

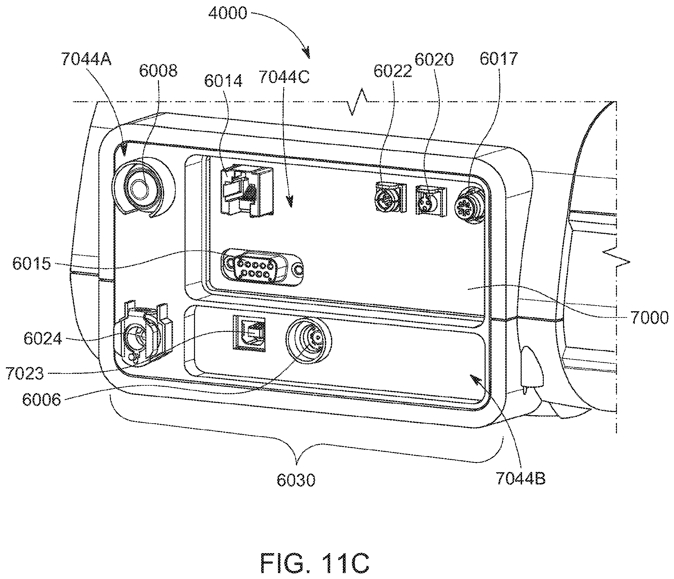

Fig. 11C illustrates a further form of the technology wherein the connectivity module 7000 is retained within the cavity using magnetic attraction and or a friction/interference fit as described herein.

As per the example of Fig. 11B the connector assembly 6030 comprises three regions 7044A-C. The first region 7044A comprises a power button 6008 and LFO₂ connector 6024. The second region 7044B comprises a DC power connection 6006 and USB-B connection 7023. The third region 7044C includes a connectivity module 7000 which provides a D-subminiature connector 6015, an ethernet connector 6014, remote alarm connection 6022, SpO₂ sensor connection 6020, and an FiO₂ sensor connection 6017.

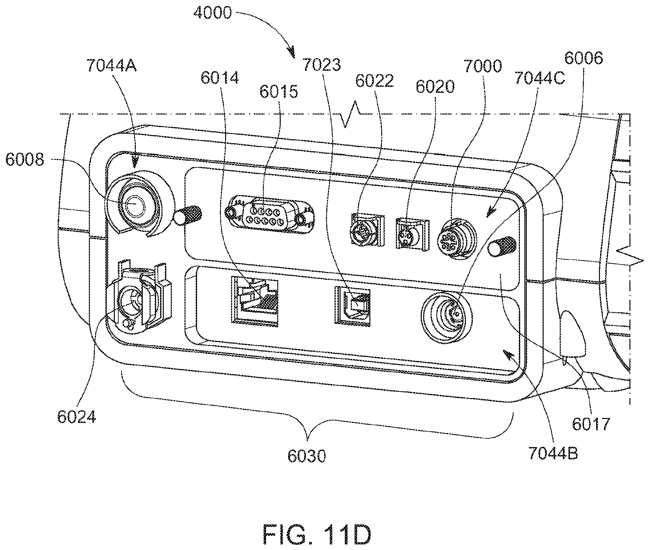

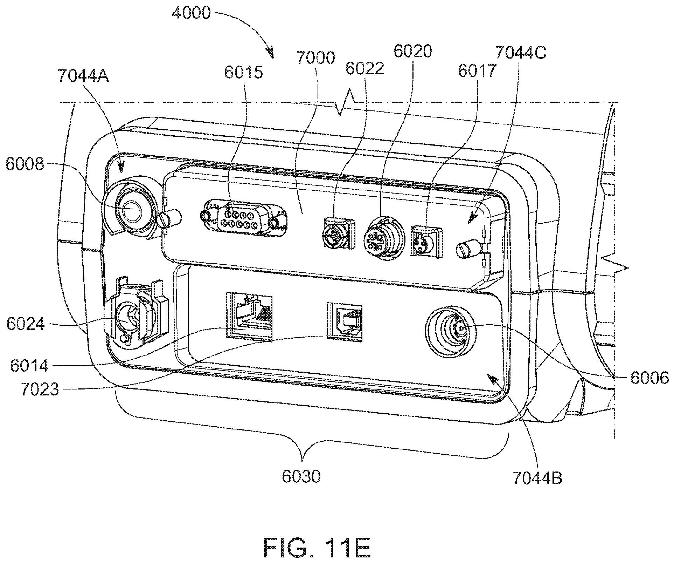

Figs. 11C-11E show examples in which the connectivity module 7000 is in the connected configuration in the cavity 7024 of the connector assembly 6030 and in which the connectivity module 7000 is: recessed into the cavity (Fig. 11C); flush with the cavity (Fig. 11D); and protruding outwardly of the cavity (Fig. 11E) i.e. only partly received by the cavity.

In the examples of Figs. 11D and 11E, three regions are provided 7044A-C. The first region 7044A comprises a power button 6008 and LFO₂ connector 6024. The second region 7044B comprises a DC power connection 6006, an ethernet connector 6014 and USB-B connection 7023. The third region 7044C includes a connectivity module 7000 which provides a D-subminiature connector 6015, remote alarm connection 6022, SpO₂ sensor connection 6020, and an FiO₂ sensor connection 6017.

### 5.7.4.2 Exemplary Connector Assemblies

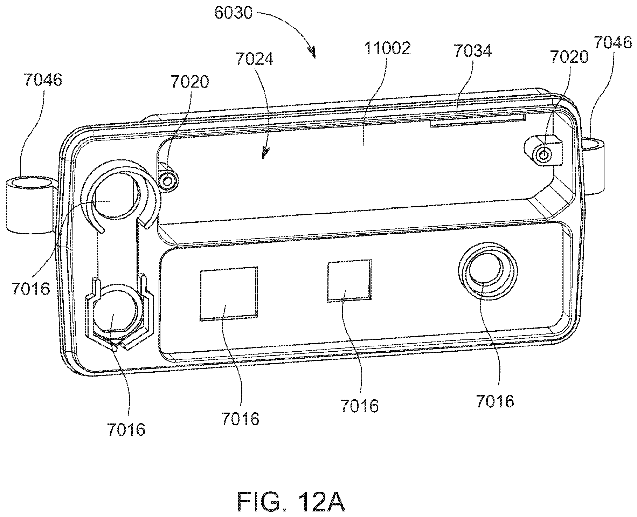

Figs. 12A-12C show one form of a connector assembly 6030 with the electrical connectors 6002 removed. As shown the connector assembly 6030 comprises a panel 11002 or section of the housing 4010 which is removed from the rest of the housing 4010. As previously discussed, providing a separate panel 1102 for the connector assembly 6030 may have numerous benefits relating to the manufacture of an RPT device 4000.

The connector assembly 6030 comprises attachment mechanisms 7020 in the form of threaded inserts, a slot 7034 configured to receive an interfacing connector 7018 and apertures 7016 configured to receive electrical connectors 6002, mechanical connectors and buttons.

Also provided are mounting posts 7046 for securing the connector assembly 6030 to the chassis or body of the RPT device 4000.

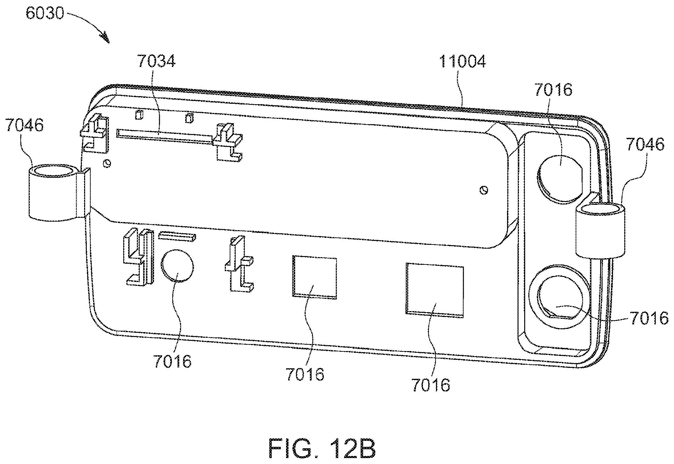

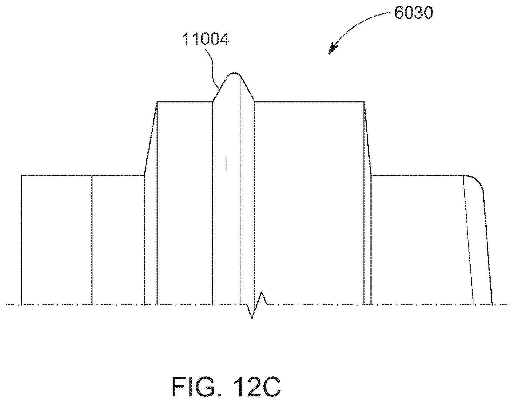

Fig. 12C provides a side view of part of the connector assembly 6030 of Figs. 12A and 12B. In this view it can be seen that the connector assembly 6030 is provided with a radial seal 11004 which extends outwardly around a perimeter of the connector assembly. This radial seal may advantageously allow the connector assembly to seal with the cavity 7024 or housing 4010 of the RPT device in use. For example, the radial seal may be positioned within a groove or channel (not shown) in the housing 4010 in order to provide a measure of ingress protection.

### 5.7.5 Connectivity Systems

According to one form of the present technology, there are provided connectivity systems comprising a least one RPT device 4000 and at least one connectivity module 7000.

In one form the of the connectivity system, an RPT device 4000 may be provided with a plurality of connectivity modules 7000 that can be interchangeably connected to the RPT device 4000. For example, a user may wish to have a first connectivity module installed by their bedside, and a second connectivity module which the user may use while travelling. In this way, the first connectivity module can remain connected to all necessary peripheral devices and the user simply has to detach the RPT device 4000 from the connectivity module 7000. This may be faster, more convenient and less prone to errors when compared to removing each connected cable individually.

In another form of the connectivity system, an RPT device 4000 may be provided with one of a plurality of connectivity modules 7000 where each of the connectivity modules 7000 comprises a different set of electrical connectors 6002. For example, a user may be provided with a connectivity module which only contains the connectors they intend to, or need to, use. Some of the electrical connectors 6002 may be common to the sets of electrical connectors 6002 for each connectivity module 7000. This potentially reduces cost for the customer, as they are not required to pay for connections they do not intend to use, as well as reducing the overall unit complexity. Conversely, a more experienced user, or a user who uses more features of the device may use a more fully featured connectivity module in order to get access to all features required.

According to a further form of a connectivity system, a service centre may be provided with a plurality of connectivity modules which can be swapped as necessary when servicing RPT devices.

According to a further form of a connectivity system, an RPT device may be provided without a removable connectivity module 7000 and the base functionality may be provided by fixed connectors on the connector assembly. In this form, connectivity modules 7000 may be provided separately, for example as an upgrade, in order to provide additional functionality. Where functionality is associated with the connectivity modules 7000 provided, it may be beneficial for the RPT device 4000 to detect the presence and type of connectivity module in order to enable and/or disable software features accordingly. Accordingly, in certain forms of the technology, the connectivity module 7000 comprises an identifier and the RPT device 4000 comprises an identifying device configured to identify the identifier on the connectivity module 7000. The identifying device of the RPT device 4000 may be configured to communicate the identity of the identified connectivity module 7000 to the central controller 4230 or other processor on the RPT device 4000, which may be configured to adjust the functioning of the RPT device 4000 accordingly.

According to a further form of a connectivity system, a single connectivity module 7000 may be configured to operate with a plurality of RPT devices 4000. For example, a common connectivity module 7000 may be configured to work with a plurality of different RPT devices 4000, such as ventilators, humidifiers and monitoring equipment. Additionally, or alternatively, a single connectivity module 7000 may be configured to operate with multiple models of RPT device, for example newer models of RPT device 4000 may be compatible with older connectivity modules 7000.

### 5.7.6 Connectivity Methods

According to one form of the present technology, there is provided a method of attaching a connectivity module 7000 to an RPT device 4000 comprising the steps of: inserting the connectivity module 7000 into a cavity in the RPT device 4000, and optionally securing the connectivity module 7000 in the cavity 7024 using an attachment mechanism 7020.

According to one form of the present technology, there is provided a method of removing a connectivity module 7000 from an RPT device 4000 comprising the steps of: optionally releasing the attachment mechanisms 7020 and removing the connectivity module from the cavity 7024 in the RPT device 4000.

### 5.8 SCREENING, DIAGNOSIS, MONITORING SYSTEMS

### 5.8.1 Polysomnography

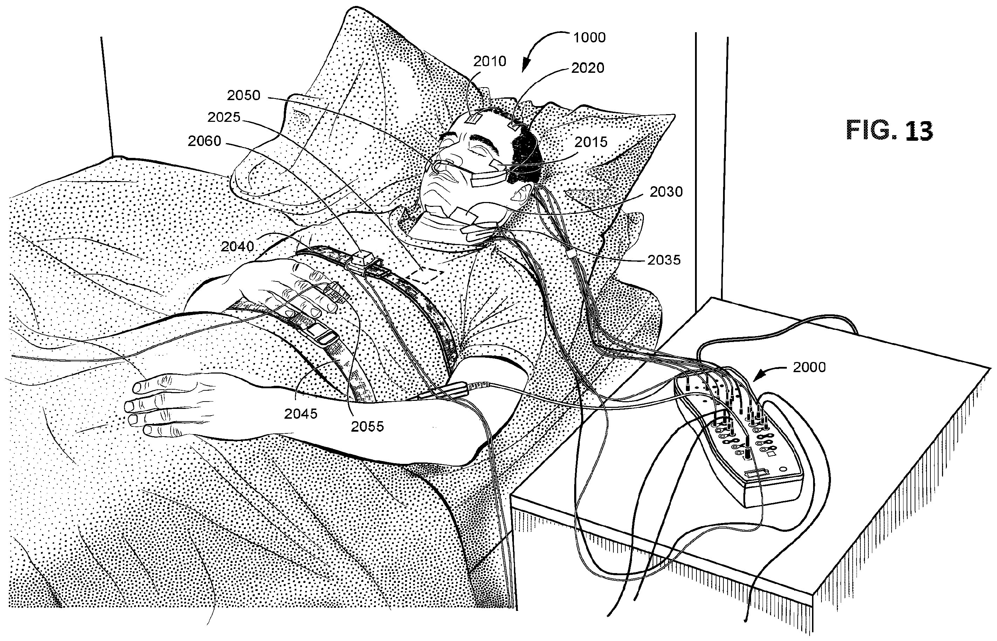

Fig. 13 shows a patient 1000 undergoing polysomnography (PSG). A PSG system comprises a headbox 2000 which receives and records signals from the following sensors: an EOG electrode 2015; an EEG electrode 2020; an ECG electrode 2025; a submental EMG electrode 2030; a snore sensor 2035; a respiratory inductance plethysmogram (respiratory effort sensor) 2040 on a chest band; a respiratory inductance plethysmogram (respiratory effort sensor) 2045 on an abdominal band; an oro-nasal cannula 2050 with oral thermistor; a photoplethysmograph (pulse oximeter) 2055; and a body position sensor 2060. The electrical signals are referred to a ground electrode (ISOG) 2010 positioned in the centre of the forehead.

In accordance with the present technology, it may be advantageous to provide the headbox with a connectivity module 7000 containing the plurality of sensors. This may advantageously allow the sensors to be decoupled from the PSG system. One advantage of this approach is that it may allow for a single PSG system to be used across multiple patients without disconnecting the plurality of sensors. For example, each patient may be wired with a plurality of sensors which are connected to a connectivity module 7000. The PSG system can then be moved between patients by simply connecting it to each of the connectivity modules in turn.

### 5.9 GLOSSARY

For the purposes of the present technology disclosure, in certain forms of the present technology, one or more of the following definitions may apply. In other forms of the present technology, alternative definitions may apply.

### 5.9.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.9.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft" materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.9.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened:* Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped:* Having a single local peak, the peak being at the leading edge, followed by a relatively flat portion.
(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.9.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

*Inspiratory positive airway pressure (IPAP):* Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS = IPAP - EPAP*)*.* In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous*/*Timed (S*/*I):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.9.4 Anatomy

### 5.9.4.1 Anatomy of the face

*Ala:* the external outer wall or "wing" of each nostril (plural: alar)

### Alar angle:

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasalpoint:* Located on the soft tissue, the point at which the columella merges with the upper lip in the midsagittal plane.

*Supramenton:* The point of greatest concavity in the midline of the lower lip between labrale inferius and soft tissue pogonion

### 5.9.4.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.9.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.9.5 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example, the headgear may comprise a collection of one or more struts. ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

## Claims

1. A system for providing respiratory therapy, the system comprising:
an apparatus (4000) for supplying a flow of breathable gas at a positive pressure for respiratory therapy;
a first removable connectivity module (7000); and
a second removable connectivity module (7000),
wherein the apparatus (4000) comprises:
a pressure generator (4140) for generating the flow of breathable gas and supplying the flow to an outlet (5004);
a housing (4010) which contains at least the pressure generator (4140); and
at least one electrical connector (6002),
wherein the housing (4010) comprises a cavity (7024) configured to interchangeably receive the first removable connectivity module (7000) and the second removable connectivity module (7000), and
wherein the at least one electrical connector (6002) is configured to interchangeably connect to the first removable connectivity module (7000) and the second removable connectivity module (7000) in use,
wherein each of the first removable connectivity module (7000) and the second removable connectivity module (7000) comprises:
a housing (7002) which comprises a front side (7004) and a rear side (7006); and
at least one electrical connector (6002) on the front side (7004), and at least one electrical connector (7018) on the rear side (7006);
wherein the housing is configured to be at least partially inserted into the cavity (7024) of the apparatus (4000) in use, and
wherein the at least one electrical connector (7018) on the rear side (7006) is configured to connect to the at least one electrical connector (6002) of the apparatus,
wherein the first removable connectivity module (7000) comprises a first set of electrical connectors (6002) and the second removable connectivity module (7000) comprises a second set of electrical connectors (6002), wherein the first set of electrical connectors (6002) is different to the second set of electrical connectors (6002).

2. The system as claimed in claim 1, wherein the cavity (7024) is located in a rear of the housing (4010) of the apparatus (4000).

3. The system as claimed in claim 1 or 2, wherein the cavity (7024) comprises one or more indexing features (7022) to ensure that each connectivity module (7000) can only be inserted into the cavity (7024) in a single orientation, and wherein preferably the indexing features (7022) comprises a chamfered corner of the cavity (7024).

4. The system of any one of the preceding claims, wherein the system further comprises at least one attachment mechanism (7020) configured to selectively secure each connectivity module (7000) at least partially within the cavity (7024), and wherein the attachment mechanism (7020) preferably comprises one or more of clips, fasteners, protrusions, apertures or magnets.

5. The system of any one of the preceding claims, further comprising a connector assembly (6030), wherein each connectivity module (7000) is configured to interchangeably connect, in use, to the connector assembly (6030), wherein the connector assembly (6030) is fixed to the housing (4010), and wherein the cavity (7024) is provided in the connector assembly (6030).

6. The system of claim 5, wherein the connector assembly (6030) comprises at least one power connector (6006).

7. The system as claimed in any one of the preceding claims, wherein the cavity (7024) comprises a slot (7038) configured to receive the at least one electrical connector (7018) on the rear side (7006), wherein the at least one electrical connector (7018) on the rear side (7006) passes through the slot (7038) and connects to the at least one electrical connector (6002) of the apparatus (4000).

8. The system of any one of the preceding claims, wherein the cavity (7024) has one or more of: a depth of between approximately 15mm and approximately 35mm, a height of between approximately 50mm and approximately 80mm, and a width of between approximately 75mm and approximately 100mm.

9. The system of any one of the preceding claims, wherein the apparatus (4000) comprises a protective cover (6010) configured to cover the at least one electrical connector (6002) of the apparatus (4000) in use.

10. The system of any one of the preceding claims, wherein the at least one electrical connector (6002) of the apparatus (4000) comprises:
at least one communication connector (6004) to facilitate the transfer of information to or from the apparatus (4000), and wherein the at least one communication connector (6004) preferably comprises any one or more communication connectors selected from the group consisting of: D-subminiature connector; USB connector; and ethernet connector, and/or
at least one power connector (6006) to facilitate the transfer of power to or from the apparatus (4000) wherein the at least one power connector preferably comprises any one or more power connectors selected from the group consisting of: DC connector; AC connector; USB connector; and ethernet connector.

11. The system of any one of the preceding claims, wherein the at least one electrical connector (6002) on the front side (7004) of one or more of the first removable connectivity module (7000) and the second removably connectivity module (7000) comprises at least one of: a USB connector, a d-subminiature connector, an ethernet connector, an SpO2 sensor connector and/or a remote alarm connector.

12. The system of any one of the preceding claims, further comprising a protective cover (6010) configured to selectively cover the at least one electrical connector (6002) on the front side of each connectivity module (7000).

13. The system of any one of the preceding claims, wherein the housing of each connectivity module comprises a shell and a backplate, and wherein preferably the shell is ultrasonically welded to the backplate.

14. The system of any one of the preceding claims, wherein each of the connectivity modules further comprises an electronic printed circuit board (PCB), and wherein the PCB comprises one or more of edge contacts, an output device in the form of a light emitting diode, and/or one or more identification components which enable the apparatus to detect and/or identify the respective connectivity module.

15. The system of any one of the preceding claims, wherein each of the modules has one or more of: a thickness of between approximately 15mm and approximately 35mm, a height of between approximately 50mm and approximately 80mm, and a width of between approximately 75mm and approximately 100mm.

## Patentansprüche

1. System zur Bereitstellung von Atemtherapie, wobei das System aufweist:
eine Vorrichtung (4000) zum Zuführen einer Atemgasströmung mit einem Überdruck zur Atemtherapie,
ein erstes entfernbares Konnektivitätsmodul (7000); und
ein zweites entfernbares Konnektivitätsmodul (7000),
wobei die Vorrichtung (4000) aufweist:
einen Druckgenerator (4140) zum Erzeugen der Atemgasströmung und Zuführen der Strömung zu einem Auslass (5004);
ein Gehäuse (4010), das zumindest den Druckgenerator (4140) enthält; und mindestens einen elektrischen Verbinder (6002),
wobei das Gehäuse (4010) einen Hohlraum (7024) aufweist, der so konfiguriert ist, dass er das erste entfernbare Konnektivitätsmodul (7000) und das zweite entfernbare Konnektivitätsmodul (7000) austauschbar aufnimmt, und
wobei der mindestens eine elektrische Verbinder (6002) so konfiguriert ist, dass er mit dem ersten entfernbaren Konnektivitätsmodul (7000) und dem zweiten entfernbaren Konnektivitätsmodul (7000) im Gebrauch austauschbar verbunden ist,
wobei das erste entfernbare Konnektivitätsmodul (7000) und das zweite entfernbare Konnektivitätsmodul (7000) jeweils aufweisen:
ein Gehäuse (7002), das eine Vorderseite (7004) und eine Rückseite (7006) aufweist; und
mindestens einen elektrischen Verbinder (6002) auf der Vorderseite (7004) und mindestens einen elektrischen Verbinder (7018) auf der Rückseite (7006);
wobei das Gehäuse so konfiguriert ist, dass es zumindest teilweise in den Hohlraum (7024) der Vorrichtung (4000) im Gebrauch eingesetzt ist, und
wobei der mindestens eine elektrische Verbinder (7018) auf der Rückseite (7006) so konfiguriert ist, dass er mit dem mindestens einen elektrischen Verbinder (6002) der Vorrichtung verbunden ist,
wobei das erste entfernbare Konnektivitätsmodul (7000) einen ersten Satz elektrischer Verbinder (6002) aufweist und das zweite entfernbare Konnektivitätsmodul (7000) einen zweiten Satz elektrischer Verbinder (6002) aufweist, wobei sich der erste Satz elektrischer Verbinder (6002) vom zweiten Satz elektrischer Verbinder (6002) unterscheidet.

2. System nach Anspruch 1, wobei der Hohlraum (7024) in einer Rückseite des Gehäuses (4010) der Vorrichtung (4000) liegt.

3. System nach Anspruch 1 oder 2, wobei der Hohlraum (7024) ein oder mehrere Bezugsmerkmale (7022) aufweist, um zu gewährleisten, dass jedes Konnektivitätsmodul (7000) in den Hohlraum (7024) nur in einer einzigen Orientierung eingesetzt werden kann, und wobei die Bezugsmerkmale (7022) vorzugsweise eine abgefaste Ecke des Hohlraums (7024) aufweisen.

4. System nach einem der vorstehenden Ansprüche, wobei das System ferner mindestens einen Befestigungsmechanismus (7020) aufweist, der so konfiguriert ist, dass er jedes Konnektivitätsmodul (7000) zumindest teilweise im Hohlraum (7024) selektiv befestigt, und wobei der Befestigungsmechanismus (7020) vorzugsweise Clips, Befestigungselemente, Vorsprünge, Öffnungen und/oder Magnete aufweist.

5. System nach einem der vorstehenden Ansprüche, ferner mit einer Verbinderanordnung (6030), wobei jedes Konnektivitätsmodul (7000) so konfiguriert ist, dass es im Gebrauch mit der Verbinderanordnung (6030) austauschbar verbunden ist, wobei die Verbinderanordnung (6030) am Gehäuse (4010) fixiert ist und wobei der Hohlraum (7024) in der Verbinderanordnung (6030) vorgesehen ist.

6. System nach Anspruch 5, wobei die Verbinderanordnung (6030) mindestens einen Stromverbinder (6006) aufweist.

7. System nach einem der vorstehenden Ansprüche, wobei der Hohlraum (7024) einen Schlitz (7038) aufweist, der so konfiguriert ist, dass er den mindestens einen elektrischen Verbinder (7018) auf der Rückseite (7006) aufnimmt, wobei der mindestens eine elektrische Verbinder (7018) auf der Rückseite (7006) den Schlitz (7038) durchläuft und mit dem mindestens einen elektrischen Verbinder (6002) der Vorrichtung (4000) verbunden ist.

8. System nach einem der vorstehenden Ansprüche, wobei der Hohlraum (7024) eine Tiefe zwischen etwa 15 mm und etwa 35 mm, eine Höhe zwischen etwa 50 mm und etwa 80 mm und/oder eine Breite zwischen etwa 75 mm und etwa 100 mm hat.

9. System nach einem der vorstehenden Ansprüche, wobei die Vorrichtung (4000) eine Schutzabdeckung (6010) aufweist, die so konfiguriert ist, dass sie den mindestens einen elektrischen Verbinder (6002) der Vorrichtung (4000) im Gebrauch abdeckt.

10. System nach einem der vorstehenden Ansprüche, wobei der mindestens eine elektrische Verbinder (6002) der Vorrichtung (4000) aufweist:
mindestens einen Kommunikationsverbinder (6004), um die Informationsübertragung zu oder von der Vorrichtung (4000) zu erleichtern, und wobei der mindestens eine Kommunikationsverbinder (6004) vorzugsweise einen oder mehrere Kommunikationsverbinder aufweist, die aus der Gruppe ausgewählt sind, die aus einem D-Subminiaturverbinder; USB-Verbinder; und Ethernet-Verbinder besteht, und/oder
mindestens einen Stromverbinder (6006), um die Stromübertragung zu oder von der Vorrichtung (4000) zu erleichtern, wobei der mindestens eine Stromverbinder vorzugsweise einen oder mehrere Stromverbinder aufweist, die aus der Gruppe ausgewählt sind, die aus einem DC-Verbinder; AC-Verbinder; USB-Verbinder; und Ethernet-Verbinder besteht.

11. System nach einem der vorstehenden Ansprüche, wobei der mindestens eine elektrische Verbinder (6002) auf der Vorderseite (7004) des ersten entfernbaren Konnektivitätsmoduls (7000) und/oder des zweiten entfernbaren Konnektivitätsmoduls (7000) aufweist: einen USB-Verbinder, einen D-Subminiaturverbinder, einen Ethernet-Verbinder, einen SpO2-Sensorverbinder und/oder einen Fernalarmverbinder.

12. System nach einem der vorstehenden Ansprüche, ferner mit einer Schutzabdeckung (6010), die so konfiguriert ist, dass sie den mindestens einen elektrischen Verbinder (6002) auf der Vorderseite jedes Konnektivitätsmoduls (7000) abdeckt.

13. System nach einem der vorstehenden Ansprüche, wobei das Gehäuse jedes Konnektivitätsmoduls einen Mantel und eine Trägerplatte aufweist und wobei vorzugsweise der Mantel mit der Trägerplatte ultraschallverschweißt ist.

14. System nach einem der vorstehenden Ansprüche, wobei jedes der Konnektivitätsmodule ferner eine elektronische Leiterplatte (PCB) aufweist und wobei die PCB Kantenkontakte, ein Ausgabebauelement in Form einer Leuchtdiode und/oder mehrere Identifizierungskomponenten aufweist, mit denen die Vorrichtung das jeweilige Konnektivitätsmodul detektieren und/oder identifizieren kann.

15. System nach einem der vorstehenden Ansprüche, wobei jedes der Module ferner eine Dicke zwischen etwa 15 mm und etwa 35 mm, eine Höhe zwischen etwa 50 mm und etwa 80 mm und/oder eine Breite zwischen etwa 75 mm und etwa 100 mm hat.

## Revendications

1. Système de fourniture de thérapie respiratoire, le système comprenant :
un appareil (4000) destiné à fournir un flux de gaz pouvant être respiré à une pression positive à des fins de thérapie respiratoire ;
un premier module de connectivité amovible (7000) ; et
un second module de connectivité amovible (7000),
dans lequel l'appareil (4000) comprend :
un générateur de pression (4140) destiné à générer le flux de gaz pouvant être respiré et à fournir le flux à un orifice de sortie (5004) ;
un boîtier (4010) qui contient au moins le générateur de pression (4140) ; et
au moins un connecteur électrique (6002),
dans lequel le boîtier (4010) comprend une cavité (7024) configurée pour recevoir de manière interchangeable le premier module de connectivité amovible (7000) et le second module de connectivité amovible (7000), et
dans lequel l'au moins un connecteur électrique (6002) est configuré pour être connecté de manière interchangeable au premier module de connectivité amovible (7000) et au second module de connectivité amovible (7000) en utilisation,
dans lequel chacun du premier module de connectivité amovible (7000) et du second module de connectivité amovible (7000) comprend :
un boîtier (7002) qui comprend un côté avant (7004) et un côté arrière (7006) ; et
au moins un connecteur électrique (6002) du côté avant (7004), et au moins un connecteur électrique (7018) du côté arrière (7006) ;
dans lequel le boîtier est configuré pour être au moins partiellement inséré dans la cavité (7024) de l'appareil (4000) en utilisation, et
dans lequel l'au moins un connecteur électrique (7018) du côté arrière (7006) est configuré pour être connecté à l'au moins un connecteur électrique (6002) de l'appareil,
dans lequel le premier module de connectivité amovible (7000) comprend un premier ensemble de connecteurs électriques (6002) et le second module de connectivité amovible (7000) comprend un second ensemble de connecteurs électriques (6002), dans lequel le premier ensemble de connecteurs électriques (6002) est différent du second ensemble de connecteurs électriques (6002).

2. Système selon la revendication 1, dans lequel la cavité (7024) est située dans une partie arrière du boîtier (4010) de l'appareil (4000).

3. Système selon la revendication 1 ou la revendication 2, dans lequel la cavité (7024) comprend une ou plusieurs caractéristiques d'indexage (7022) pour garantir le fait de chaque module de connectivité (7000) ne puisse être inséré dans la cavité (7024) que dans une seule orientation, et dans lequel, de préférence, les caractéristiques d'indexage (7022) comprennent un coin chanfreiné de la cavité (7024).

4. Système selon l'une quelconque des revendications précédentes, dans lequel le système comprend en outre au moins un mécanisme de fixation (7020) configuré pour bloquer sélectivement chaque module de connectivité (7000) au moins partiellement à l'intérieur de la cavité (7024), et dans lequel le mécanisme de fixation (7020) comprend de préférence un ou plusieurs éléments parmi des clips, des attaches, des saillies, des ouvertures ou des aimants.

5. Système selon l'une quelconque des revendications précédentes, comprenant en outre un ensemble de raccordement (6030), dans lequel chaque module de connectivité (7000) est configuré pour être connecté de manière interchangeable, en utilisation, à l'ensemble de raccordement (6030), dans lequel l'ensemble de raccordement (6030) est fixé au boîtier (4010), et dans lequel la cavité (7024) est prévue dans l'ensemble de raccordement (6030).

6. Système selon la revendication 5, dans lequel l'ensemble de raccordement (6030) comprend au moins un connecteur d'alimentation(6006).

7. Système selon l'une quelconque des revendications précédentes, dans lequel la cavité (7024) comprend une fente (7038) configurée pour recevoir l'au moins un connecteur électrique (7018) du côté arrière (7006), dans lequel l'au moins un connecteur électrique (7018) du côté arrière (7006) passe à travers la fente (7038) et se connecte à l'au moins un connecteur électrique (6002) de l'appareil (4000).

8. Système selon l'une quelconque des revendications précédentes, dans lequel la cavité (7024) a une ou plusieurs dimensions parmi : une profondeur comprise entre environ 15 mm et environ 35 mm, une hauteur comprise entre environ 50 mm et environ 80 mm, et une largeur comprise entre environ 75 mm et environ 100 mm.

9. Système selon l'une quelconque des revendications précédentes, dans lequel l'appareil (4000) comprend un couvercle de protection (6010) configuré pour couvrir l'au moins un connecteur électrique (6002) de l'appareil (4000) en utilisation.

10. Système selon l'une quelconque des revendications précédentes, dans lequel l'au moins un connecteur électrique (6002) de l'appareil (4000) comprend :
au moins un connecteur de communication (6004) servant à faciliter le transfert d'informations vers l'appareil (4000) ou à partir de ce dernier, et dans lequel l'au moins un connecteur de communication (6004) comprend de préférence un ou plusieurs connecteurs de communication sélectionnés dans le groupe constitué : d'un connecteur subminiature D, d'un connecteur USB ; et d'un connecteur Ethernet, et/ou
au moins un connecteur électrique (6006) servant à faciliter le transfert d'électricité vers l'appareil (4000) ou à partir de ce dernier, dans lequel l'au moins un connecteur électrique comprend de préférence un ou plusieurs connecteurs électriques sélectionnés dans le groupe constitué : d'un connecteur CC ; d'un connecteur CA ; d'un connecteur USB ; et d'un connecteur Ethernet.

11. Système selon l'une quelconque des revendications précédentes, dans lequel l'au moins un connecteur électrique (6002) du côté avant (7004) d'un ou des modules du premier module de connectivité amovible (7000) et du second module de connectivité amovible (7000) comprend au moins l'un parmi : un connecteur USB, un connecteur subminiature d, un connecteur Ethernet, un connecteur de capteur de SpO2 et/ou un connecteur de téléalarme.

12. Système selon l'une quelconque des revendications précédentes, comprenant en outre un couvercle de protection (6010) configuré pour couvrir sélectivement l'au moins un connecteur électrique (6002) du côté avant de chaque module de connectivité (7000).

13. Système selon l'une quelconque des revendications précédentes, dans lequel le boîtier de chaque module de connectivité comprend une coque et une plaque arrière, et dans lequel, de préférence, la coque est soudée par ultrasons à la plaque arrière.

14. Système selon l'une quelconque des revendications précédentes, dans lequel chacun des modules de connectivité comprend en outre une carte de circuits imprimés (PCB) électronique, et dans lequel la PCB comprend un ou plusieurs contacts d'enfichage, un dispositif de sortie sous la forme d'une diode électroluminescente, et/ou un ou plusieurs composants d'identification qui permettent à l'appareil de détecter et/ou d'identifier le module de connectivité respectif.

15. Système selon l'une quelconque des revendications précédentes, dans lequel chacun des modules a une ou plusieurs dimensions parmi : une épaisseur comprise entre environ 15 mm et environ 35 mm, une hauteur comprise entre environ 50 mm et environ 80 mm, et une largeur comprise entre environ 75 mm et environ 100 mm.
